# EUROPÄISCHE PATENTSCHRIFT

(11) **EP 1 239 817 B1**
(45) Veröffentlichungstag und Bekanntmachung des Hinweises auf die Patenterteilung: **19.04.2006**
(21) Anmeldenummer: 00990772.6
(22) Anmeldetag: 15.12.2000
(51) Int. Cl.: C07D 215/14, A61Q 5/10, A61K 8/49, A61K 8/41

(54) **VERWENDUNG VON METHYLCHINOLINIUM-VERBINDUNGEN ZUM FÄRBEN VON KERATINHALTIGEN FASERN**
USE OF METHYLCHINOLINIUM COMPOUNDS FOR DYEING KERATIN CONTAINING FIBERS
UTILISATION DE COMPOSES DE METHYLQUINOLINIUM POUR COLORER DES FIBRES KERATINIQUES

(30) Priorität: 24.12.1999 DE 19962875
(43) Veröffentlichungstag der Anmeldung: 18.09.2002
(73) Patentinhaber: Henkel Kommanditgesellschaft auf Aktien, 40589 Düsseldorf (DE)
(72) Erfinder: MÖLLER, Hinrich, 40789 Monheim (DE); OBERKOBUSCH, Doris, 40591 Düsseldorf (DE); HÖFFKES, Horst, 40595 Düsseldorf (DE)
(86) Internationale Anmeldenummer: PCT/EP2000/012816
(87) Internationale Veröffentlichungsnummer: WO 2001/047483

(56) Entgegenhaltungen:
- WO-A-00/61091
- WO-A-01/10379
- DE-A- 19 745 356
- DE-U- 29 908 573

## Beschreibung

Die Erfindung betrifft die Verwendung von Formyl-1-methylchinolinium-Verbindungen zum Färben von keratinhaltigen Fasern, insbesondere menschlichen Haaren, ein Mittel zum Färben von keratinhaltigen Fasern, das Formyl-1-methylchinolinium-Verbindungen enthält, sowie ein Verfahren zum Färben von keratinhaltigen Fasern.

Für das Färben von keratinhaltigen Fasern, z. B. Haaren, Wolle oder Pelzen, kommen im allgemeinen entweder direktziehende Farbstoffe oder Oxidationsfarbstoffe, die durch oxidative Kupplung einer oder mehrerer Entwicklerkomponenten untereinander oder mit einer oder mehreren Kupplerkomponenten entstehen, zur Anwendung. Kuppler- und Entwicklerkomponenten werden auch als Oxidationsfarbstoffvorprodukte bezeichnet.

Als Entwicklerkomponenten werden üblicherweise primäre aromatische Amine mit einer weiteren, in para- oder ortho-Position befindlichen freien oder substituierten Hydroxy- oder Aminogruppe, Diaminopyridinderivate, heterocyclische Hydrazone, 4-Aminopyrazolonderivate sowie 2,4,5,6-Tetraaminopyrimidin und dessen Derivate eingesetzt.

Spezielle Vertreter sind beispielsweise p-Phenylendiamin, p-Toluylendiamin, 2,4,5,6-Tetraaminopyrimidin, p-Aminophenol, N,N-Bis-(2-hydroxyethyl)-p-phenylendiamin, 2-(2,5-Diaminophenyl)-ethanol, 2-(2,5-Diaminophenoxy)-ethanol, 1-Phenyl-3-carboxyamido-4-amino-pyrazolon-5, 4-Amino-3-methylphenol, 2-Aminomethyl-4-aminophenol, 2-Hydroxymethyl-4-aminophenol, 2-Hydroxy-4,5,6-triaminopyrimidin, 2,4-Dihydroxy-5,6-diaminopyrimidin und 2,5,6-Triamino-4-hydroxypyrimidin.

Als Kupplerkomponenten werden in der Regel m-Phenylendiaminderivate, Naphthole, Resorcin und Resorcinderivate, Pyrazolone und m-Aminophenole verwendet. Als Kupplersubstanzen eignen sich insbesondere α-Naphthol, 1,5-, 2,7- und 1,7-Dihydroxynaphthalin, 5-Amino-2-methylphenol, m-Aminophenol, Resorcin, Resorcinmonomethylether, m-Phenylendiamin, 2,4-Diaminophenoxyethanol, 1-Phenyl-3-methyl-pyrazolon-5, 2,4-Dichlor-3-aminophenol, 1,3-Bis-(2,4-diaminophenoxy)-propan, 2-Chlorresorcin, 4-Chlorresorcin, 2-Chlor-6-methyl-3-aminophenol, 2-Methylresorcin und 5-Methylresorcin.

Bezüglich weiterer üblicher Farbstoffkomponenten wird ausdrücklich auf die Reihe "Dermatology", herausgeben von Ch. Culnan, H. Maibach, Verlag Marcel Dekker lnc., New York, Basel, 1986, Bd. 7, Ch. Zviak, The Science of Hair Care, Kap. 7, Seiten 248 - 250 (Direktziehende Farbstoffe) und Kap. 8, Seiten 264 - 267 (Oxidationsfarbstoffe) sowie das "Europäische Inventar der Kosmetikrohstoffe", 1996, herausgegeben von der Europäischen Kommission, erhältlich in Diskettenform vom Bundesverband der deutschen Industrie- und Handelsuntemehmen für Arzneimittel, Reformwaren und Körperpflegemittel e.V., Mannheim, Bezug genommen.

Mit Oxidationsfarbstoffen lassen sich zwar intensive Färbungen mit guten Echtheitseigenschaften erzielen, die Entwicklung der Farbe geschieht jedoch i.a. unter dem Einfluß von Oxidationsmitteln wie z.B. H₂O₂, was in einigen Fällen Schädigungen der Faser zur Folge haben kann. Desweiteren können einige Oxidationsfarbstoffvorprodukte bzw. bestimmte Mischungen von Oxidationsfarbstoffvorprodukten bisweilen bei Personen mit empfindlicher Haut sensibilisierend wirken. Direktziehende Farbstoffe werden unter schonenderen Bedingungen appliziert, ihr Nachteil liegt jedoch darin, daß die Färbungen häufig nur über unzureichende Echtheitseigenschaften verfügen.

Aufgabe der vorliegenden Erfindung ist es, Färbemittel für Keratinfasem, insbesondere menschliche Haare, bereitzustellen, die hinsichtlich der Farbtiefe, der Grauabdeckung und den Echtheitseigenschaften qualitativ im Vergleich mit üblichen Oxidationshaarfärbemitteln mindestens gleichwertig sind, ohne jedoch unbedingt auf Oxidationsmittel wie z.B. H₂O₂ angewiesen zu sein. Eine weitere Aufgabe der vorliegenden Erfindung besteht darin, Färbemittel bereitzustellen, mit denen eine große Vielfalt in den Farbnuancen erhalten werden kann. Eine Anfärbung der Hautpartien sollte möglichst vermieden werden. Darüber hinaus dürfen die Färbemittel kein oder lediglich ein sehr geringes Sensibilisierungspotential aufweisen.

In der deutschen Patentanmeldung 19745356.2 wird die Verwendung von Oniumaldehyden und -ketonen zum Färben von keratinhaltigen Fasern offenbart, die in Kombination mit Verbindungen mit primärer oder sekundärer Aminogruppe oder Hydroxygruppe, ausgewählt aus primären oder sekundären aromatischen Aminen, stickstoffhaltigen heterocyclischen Verbindungen und aromatischen Hydroxyverbindungen, und/oder CH-aktiven Verbindungen eingesetzt werden.

Überraschenderweise wurde gefunden, daß spezielle Formyl-Methylchinolinium-Verbindungen der in der deutschen Patentanmeldung 197 45 356.2 offenbarten Oniumaldehyde und -ketone, wie sie nachstehend definiert werden, sich besonders gut zum Färben von keratinhaltigen Fasern eignen. Diese Verbindungen fallen in kristalliner Form an, so daß in der für Haarfärbemittel erforderliche Reinheit erhalten werden können und sich gut handhaben lassen. Es werden Ausfärbungen mit hervorragender Brillanz und Farbtiefe in vielfältigen Farbnuancen erzielt. Der Einsatz von oxidierenden Agentien ist nicht erforderlich, er soll jedoch nicht prinzipiell ausgeschlossen werden.

Gegenstand der vorliegenden Erfindung ist demgemäß die Verwendung von Formyl-1-methylchinolinium-Verbindungen mit der folgenden Formel I, 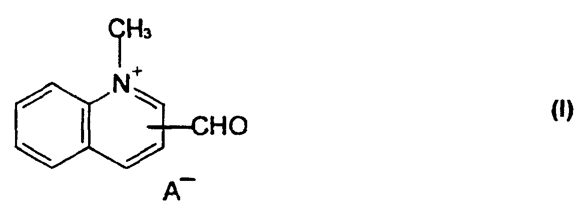 worin die Formylgruppe CHO in 2- oder 4-Position gebunden ist und A⁻ steht für ein p-Toluolsulfonat-, -lon, in Kombination mit einer weiter hinten spezifizierten ausgewählten Verbindung B
zum Färben von keratinhaltigen Fasern.

Die Verbindungen mit der Formel können auch in Form der Hydrate eingesetzt werden.

Unter keratinhaltigen Fasern sind Wolle, Pelze, Federn und insbesondere menschliche Haare zu verstehen. Die erfindungsgemäßen Färbemittel können prinzipiell aber auch zum Färben anderer Naturfasern, wie z.B. Baumwolle, Jute, Sisal, Leinen oder Seide, modifizierter Naturfasern, wie z.B. Regeneratcellulose, Nitro-, Alkyl- oder Hydroxyalkyl- oder Acetylcellulose und synthetischer Fasern, wie z.B. Polyamid-, Polyacrylnitril-, Polyurethan- und Polyesterfasern verwendet werden.

Ein weiterer Gegenstand der vorliegenden Erfindung ist ein Mittel zum Färben von keratinhaltigen Fasern, insbesondere menschlichen Haaren, das Formyl-1-methylchinolinium-Verbindungen mit der folgenden Formel I, 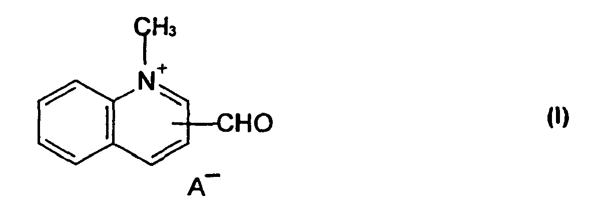 worin die Formylgruppe CHO in 2- oder 4-Position gebunden ist und A⁻ steht für ein p-Toluolsulfonat-, -Ion, und eine weiter hinten spezifizierte Verbindung B
enthält.

Die Verbindung, in der die Formylgruppe in der 2-Position gebunden ist, ist bekannt. Sie kann z. B. durch Methylierung von Chinaldin mit p-Toluolsulfonsäuremethylester, anschließende Reaktion mit N,N-Dimethyl-4-nitrosoanilin und hydrolytische Spaltung des erhaltenen Azomethins mit Salzsäure erhalten werden.

Aus der WO 01/10379, relevant gemäß Art. 54(3) EPÜ, ist die Verbindung, in der die Formylgruppe in der 4-Postion gebunden ist, bekannt.

Die Verbindungen mit der Formel 1 werden üblicherweise in einer Menge von jeweils 0,03 bis 65, insbesondere 1 bis 40 mmol, jeweils bezogen auf 100 g des gesamten Färbemittels, eingesetzt.

In allen Färbemitteln können auch mehrere verschiedene färbende Substanzen gemeinsam zum Einsatz kommen.

Färbungen mit noch erhöhter Brillanz und verbesserten Echtheitseigenschaften (Lichtechtheit, Waschechtheit, Reibechtheit) über einen weiten Nuancenbereich von gelb, orange, rot, violett, braun, bis hin zu blauschwarz und schwarz werden erzielt, wenn die erfindungsgemäß eingesetzten Verbindungen mit der Formel I gemeinsam mit mindestens einer weiteren Verbindung B (im folgenden Komponente B genannt), ausgewählt aus Verbindungen mit primärer oder sekundärer Aminogruppe oder Hydroxygruppe, ausgewählt aus weiter hinten spezifizierten primären oder sekundären aromatischen Aminen, stickstoffhaltigen heterocyclischen Verbindungen und aromatischen Hydroxyverbindungen, und/oder CH-aktiven Verbindungen, verwendet werden. Dies sind einerseits Verbindungen, die für sich alleine keratinhaltige Fasern nur schwach färben und erst gemeinsam mit den Verbindungen der Formel I brillante Färbungen ergeben. Andererseits sind darunter aber auch Verbindungen, die bereits als Oxidationsfarbstoffvorprodukte eingesetzt werden.

In allen Färbemitteln können auch mehrere verschiedene Formyl-1-methylchinolinium-Verbindungen der Formel I gemeinsam zum Einsatz kommen.

Die Formyl-1-methylchinoliniumverbindungen der Formel I in Kombination mit der Komponente B können weiterhin auch zusammen mit anderen Carbonylverbindungen eingesetzt werden. Beispiele für bevorzugte weitere Carbonylverbindungen sind:
- Isatin und Isatin-Derivate gemäß Formel (I) der WO-A1-94/24988, insbesondere der Grundkörper Isatin,
- sulfo- oder carboxylgruppenhaltige Isatin-Derivate gemäß Formel (I) der WO-A1-94/24989, insbesondere Isatin-5-sulfonsäure,
- Isatin-Derivate gemäß Formel (I) der WO-A1-95/24886, insbesondere 1-Allylisatin,
- Oniumaldehyde und -ketone gemäß WO-A2-99/18916, insbesondere 4-Formyl-1-methylpyridinium-benzolsulfonat und 4-Benzoyl-1-methyl-pyridinium-methansulfonat, sowie
- aromatische Nitroaldehyde gemäß WO-A1-00/38634, insbesondere 2,4-Dinitrobenzaldehyd.

Ebenso können auch mehrere verschiedene Verbindungen der Komponente B gemeinsam verwendet werden.

Unter die voranstehend beschriebene Ausführungsform fällt auch die Verwendung von solchen Substanzen, die Reaktionsprodukte von Formyl-1-methylchinolinium-Verbindungen der Formel I mit den genannten Verbindungen der Komponente B darstellen, als direktziehende Färbemittel. Derartige Reaktionsprodukte können z. B. durch kurzes Erwärmen der beiden Komponenten in stöchiometrischen Mengen in wäßrigem neutralen bis schwach alkalischen Milieu erhalten werden, wobei sie entweder als Feststoff aus der Lösung ausfallen oder durch Eindampfen der Lösung daraus isoliert werden. Die Reaktionsprodukte können auch in Kombination mit anderen Farbstoffen oder Farbstoffvorprodukten eingesetzt werden.

Die Komponente B wird aus
- den folgenden Verbindungen mit primärer oder sekundärer Aminogruppe oder Hydroxygruppe, ausgewählt aus primären oder sekundären aromatischen Aminen,
- den folgenden stickstoffhaltigen heterocyclischen Verbindungen,
- aromatischen Hydroxyverbindungen, und/oder
- CH-aktiven Verbindungen

Die geeigneten Verbindungen mit primärer oder sekundärer Aminogruppe als Komponente B sind die folgenden primären aromatischen Amine: N-(2-Hydroxyethyl)-N-ethyl-, N-(2-Methoxyethyl-), 2,3-, 2,4-, 2,5-Dichlor-p-phenylendiamin, 2-Chlor-p-phenylendiamin, N,N-Bis-(2-hydroxyethyl)-p-phenylendiamin, 2,5-Dihydroxy-4-morpholinoanilin-dihydrobromid, 2-, 3-, 4-Aminophenol, o-, m-, p-Phenylendiamin, 2,4-Diaminophenoxyethanol, 2-(2,5-Diaminophenyl)-ethanol, 2,5-Diaminotoluol, -phenol, -phenethol, 4-Methylamino-, 3-Amino-4-(2'-hydroxyethyloxy)-, 3,4-Methylendiamino-, 3,4-Methylendioxyanilin, 3-Amino-2,4-dichlor-, 4-Methylamino-, 2-Methyl-5-amino-, 3- Methyl-4-amino-, 2-Methyl-5-(2-hydroxyethylamino)-, 2-Methyl-5-amino-4-chlor-, 6-Methyl-3-amino-2-chlor-, 5-(2-Hydroxyethylamino)-4-methoxy-2-methyl-, 4-Amino-2-aminomethyl-phenol, 2-Hydroxymethyl-4-aminophenol, 2-Diethylaminomethyl-4-aminophenol, 2-Dimethylaminomethyl-4-aminophenol, 2,6-Dichlor-4-aminophenol, 1,3-Diamino-2,4-dimethoxybenzol, 2-, 3-, 4-Aminobenzoesäure, -phenylessigsäure, 2,3-, 2,4-, 2,5-, 3,4-, 3, 5-Diaminobenzoesäure, 4-, 5-Aminosalicylsäure, 3-Amino-4-hydroxy-, 4-Amino-3-hydroxybenzoesäure, 2-, 3-, 4-Aminobenzolsulfonsäure, 3-Amino-4-hydroxybenzolsulfonsäure, 4-Amino-3-hydroxynaphthalin-sulfonsäure, 6-Amino-7-hydroxynaphthalin-2-sulfonsäure, 7-Aniino-4-hydroxynaphthalin-2-sulfonsäure, 4-Amino-5-hydroxynaphthalin-2,7-disulfonsäure, 3-Amino-2-naphthoesäure, 3-Aminophthalsäure, 5-Aminoisophthalsäure, 1,3,5-, 1,2,4-Triaminobenzol, 1,2,4,5-Tetraaminobenzol-tetrahydrochlorid, 2,4, 5-Triaminophenol-trihydrochlorid, Pentaaminobenzol-pentahydrochlorid, Hexaaminobenzol-hexahydrochlorid, 2,4,6-Triaminoresorcin-trihydrochlorid, 4,5-Diaminobrenzcatechinsulfat, 4,6-Diaminopyrogallol-dihydrochlorid, 3,5-Diamino-4-hydroxybrenzcatechin-sulfat, 1, 3-Dimethyl-2,5-diaminobenzol, 4-Amino-1-hydroxy-2-(2'-hydroxyethylaminomethyl)-benzol, 1 -Hydroxy-2-amino-5-methyl-benzol, 1-Hydroxy-2-amino-6-methyl-benzol, 5-Amino-4-fluor-2-methylphenol 2,4-Diamino-5-fluortoluol, 2,4-Diamino-5-(2'-hydroxyethoxy)-toluol, 2,4-Diamino-5-methylphenetol, 3,5-Diamino-2-methoxy-1-methylbenzol, 2-Amino-4-(2'-hydroxyethylamino)-anisol, 2,6-Bis-(2'-hydroxyethyl)-1-methylbenzol, N,N'-Bis(4-aminophenyl)-1,4-diazacycloheptan, 1-(2-Hydroxy-5-aminobenzyl)-2-imidazolidinon, 4-Amino-2-((4-[(5-amino-2-hydroxyphenyl)methyl]-piperazinyl)methyl)phenol, 4-Nitroanilin, 4-Nitro-1,3-phenylendiamin, 4-Amino-2-nitrodiphenylamin-2'-carbonsäure, 1-Amino-5-chlor-4-(2-hydroyethylamino)-2-nitrobenzol, aromatische Aniline bzw. Phenole mit einem weiteren aromatischen Rest, wie sie in der Formel II dargestellt sind 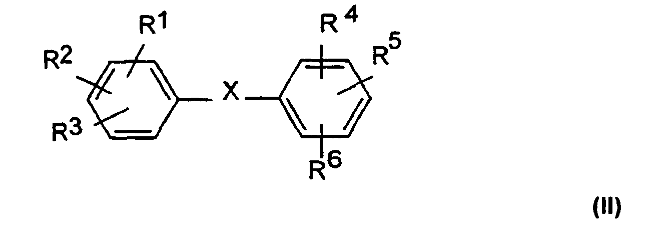 in der R¹ für eine Hydroxy- oder eine Aminogruppe, die durch C₁₋₄-Alkyl-, C₁₋₄-Hydroxyalkyl-, C₁₋₄-Alkoxy- oder C₁₋₄-Alkoxy-C₁₋₄-alkylgruppen substituiert sein kann, steht, R², R³, R⁴, R⁵ und R⁶ für Wasserstoff, eine Hydroxy- oder eine Aminogruppe, die durch C₁₋₄-Alkyl-, C₁₋₄-Hydroxyalkyl, C₁₋₄-Alkoxy-, C₁₋₄-Aminoalkyl- oder C₁₋₄-Alkoxy-C₁₋₄-alkylgruppen substituiert sein kann, stehen, und
X für eine direkte Bindung, eine gesättigte oder ungesättigte, ggf. durch Hydroxygruppen substituierte Kohlenstoffkette mit 1 bis 4 Kohlenstoffatomen, eine Carbonyl-, Sulfonyl- oder Iminogruppe, ein Sauerstoff- oder Schwefelatom, oder eine Gruppe mit der Formel III

**Z-(CH**_{**2**}**-Y-CH**_{**2**}**-CH-Z')**_{**o**} (III)

in der Y eine direkte Bindung, eine CH₂- oder CHOH-Gruppe bedeutet,
Z und Z' unabhängig voneinander für ein Sauerstoffatom, eine NR⁷-Gruppe, worin R⁸ Wasserstoff, eine C₁₋₄-Alkyl- oder eine Hydroxy-C₁₋₄-alkylgruppe bedeutet, die Gruppe O-(CH₂)ₚ-NH oder NH-(CH₂)ₚ'-O, worin p und p' 2 oder 3 sind, stehen und o eine Zahl von 1 bis 4 bedeutet,
wie beispielsweise 4,4'-Diaminostilben und dessen Hydrochlorid, 4,4'-Diaminostilben-2,2'-disulfonsäure-mono- oder -di-Na-Salz, 4-Amino-4'-dimethylaminostilben und dessen Hydrochlorind, 4,4'-Diaminodiphenylmethan, -sulfid, -sulfoxid, -amin, 4,4'-Diaminodiphenylamin-2-sulfonsäure, 4,4'-Diaminobenzophenon, -diphenylether, 3,3',4,4'-Tetraaminodiphenyl, 3,3',4,4'-Tetraamino-benzophenon, 1,3-Bis-(2,4-diaminophenoxy)-propan, 1,8-Bis-(2,5-diaminophenoxy)-3,6-dioxaoctan, 1,3-Bis-(4-aminophenylamino)propan, -2-propanol, 1,3-Bis-[N-(4-aminophenyl)-2-hydroxyethylamino]-2-propanol, N,N-Bis-[2-(4-aminophenoxy)-ethyl]-methylamin, N-Phenyl-1,4-phenylendiamin, Bis-(2-hydroxy-5-aminophenyl)-methan.

Die vorgenannten Verbindungen können sowohl in freier Form als auch in Form ihrer physiologisch verträglichen Salze, insbesondere als Salze anorganischer Säuren, wie Salz- oder Schwefelsäure, eingesetzt werden.

Die stickstoffhaltigen heterocyclischen Verbindungen werden ausgewählt aus der Gruppe bestehend aus 2-, 3-, 4-Amino-, 2-Amino-3-hydroxy-, 2,6-Diamino-, 2,5-Diamino-, 2,3-Diamino-, 2-Dimethylamino-5-amino-, 3-Amino-2-methylamino-6-methoxy-, 2,3-Diamino-6- methoxy-, 3,5-Diamino-2,6-dimethoxy-, 2,4,5-Triamino-, 2 ,6-Dihydroxy-3,4-di- methylpyridin, 4,5,6-Triamino-, 2-Hydroxy-4, 5,6-triamino-, 4-Hydroxy-2,5,6-triamino-, 2,4,5,6-Tetraamino-, 2-Methylamino-4,5,6-triamino-, 2,4-, 4,5-Diamino-, 2-Amino-4-methoxy-6-methyl-pyrimidin, 2,3,4-Trimethylpyrrol, 2,4-Dimethyl-3-ethyl-pyrrol, 3,5-Diaminopyrazol, -1,2,4-triazol, 3-Amino-, 3-Amino-5-hydroxypyrazol, 2-, 3-, 8-Aminochinolin, 4-Amino-chinaldin, 2-, 6-Aminonicotinsäure, 5-Aminoisochinolin, 5-, 6-Aminoindazol, 5-, 7-Amino-benzimidazol, -benzothiazol, 2,5-Dihydroxy-4-morpholinoanilin, 1-Phenyl-3-methyl-pyrazolon-5,4,5-Diamino-1 -(2-hydroxyethyl)-pyrazol, 2-(Aminoethylamino)-5-aminopyridin, (2((2,4-Diaminophenyl)amino)ethyl-5-amino-(2-pyridyl))amin, (2((4-Aminophenyl)amino)ethyl-5-amino-(2-pyridyl))amin sowie Indol- und Indolinderivaten, wie 4-, 5-, 6-, 7-Aminolndol, 4-, 5-, 6-, 7-Hydroxyindol, 5,6-Dihydroxyindol, 5,6-Dihydroxyindolin und 4-Hydroxyindolin, sowie jeweils aus den mit vorzugsweise anorganischen Säuren gebildeten physiologisch verträglichen Salzen dieser Verbindungen.

Geeignete aromatische Hydroxyverbindungen sind z. B. 2-, 4-, 5-Methylresorcin, 2,5-Dimethylresorcin, Resorcin, 3-Methoxyphenol, Brenzkatechin, Hydrochinon, Pyrogallol, Phloroglucin, Hydroxyhydrochinon, 2-, 3-, 4-Methoxy-, 3-Dimethylamino-, 2-(2-Hydroxyethyl)-, 3,4-Methylendioxyphenol, 2,4-, 3,4-Dihydroxybenzoesäure, -phenylessigsäure, Gallussäure, 2,4,6-Trihydroxybenzoesäure, -acetophenon, 2-, 4-Chlorresorcin, 1-Naphthol, 1,5-, 2,3-, 2,7-Dihydroxynaphthalin, 6-Dimethylamino-4-hydroxy-2-naphthalinsulfonsäure, 3,6-Dihydroxy-2,7-naphthalinsulfonsäure.

Als CH-aktive Verbindungen können beispielhaft genannt werden 1,2,3,3-Tetramethyl-3H-indoliumiodid, 1,2,3,3-Tetramethyl-3H-indolium-p-toluolsulfonat, 1,2,3,3-Tetramethyl-3H-indolium-methansulfonat, Fischersche Base (1,3,3-Trimethyl-2-methylenindolin), 2,3-Dimethyl-benzothiazoliumiodid, 2,3-Dimethyl-benzothiazolium-p-toluolsulfonat, 1,2-Dimethyl-naphtho[1,2-d]thiazolium-p-toluolsulfonat, 1-Ethyl-2-methyl-naphtho[1,2-d]thiazolium-p-toluolsulfonat, Rhodanin, Rhodanin-3-essigsäure, 1-Methyl-2-chinaldinium-iodid, 1-E-thyt-2-chinaldinium-iodid, 1,4-Dimethylchinolinium-iodid, Barbitursäure, Thiobarbitursäure, 1,3-Dimethylthiobarbitursäure, 1,3-Diethylthiobarbitursäure, Diethylthiobarbitursäure, Oxindol, 3-Indoxylacetat, Cumaranon und 1-Methyl-3-phenyl-2-pyrazolinon.

Die Verbindungen der Komponente B werden besonders bevorzugt ausgewählt aus der Gruppe bestehend aus N,N-Bis(2-hydroxyethyl)-p-phenylendiamin, 2,5-Diaminotoluol, 2-(2,5-Diaminophenyl)-ethanol, 2-Aminophenol, 3-Aminophenol, 4-Aminophenol, 2-Aminomethyl-4-aminophenol, 2-(Diethylaminomethyl)-4-aminophenol, 1-Hydroxy-2-amino-5-methyl-benzol, 2,4-Diaminophenoxyethanol, 3-Amino-2,4-dichlorphenol, 2-Methyl-5-aminophenol, 2-Methyl-5-(2-hydroxyeihylamino)-phenol, 6-Methyl-3-amino-2-chlorphenol, 2-Methyl-5-amino-4-chlorphenol, 3,4-Methylendioxyanilin, 3,4-Diaminobenzoesäure, 3.5-Diamino-2-methoxy-1-methylbenzol, 2-Amino-4-(2'-hydroxyethylamino)-anisol, 2,6-Bis-(2'-hydroxyethyl)-1-methylbenzol, 1,3-Bis-(2,4-diaminophenoxy)-propan, 4,5-Diamino-1-(2-hydroxyethyl)pyrazol, N,N'-Bis(4-aminophenyl)-1,4-diazacycloheptan, Bis-(2-hydroxy-5-aminophenyl)-methan, 1,8-Bis-(2,5-diaminophenoxy)-3,6-dioxaoctan, 4,4'-Diaminodiphenylamin, 2-Amino-3-hydroxypyridin, 3-Amino-2-methylamino-6-methoxypyridin, 2,6-Dihydroxy-3,4-dimethylpyridin, 5,6-Dihydroxyindol, 5,6-Dihydroxyindolin, 2,4,5,6-Tetraaminopyrimidin, 2-Hydroxy-4,5,6-triamino-pyrimidin, 4-Hydroxy-2,5,6-triamino-pyrimidin sowie deren mit vorzugsweise anorganischen Säuren gebildeten physiologisch verträglichen Salze.

Die voranstehend genannten Verbindungen der Komponente B können in einer Menge von jeweils 0,03 bis 65, insbesondere 1 bis 40 mmol, jeweils bezogen auf 100 g des gesamten Färbemittels, eingesetzt werden. Weiterhin kann es erfindungsgemäß besonders bevorzugt sein, die Verbindung der Formel I und die Verbindung der Komponente B im molaren Mengenverhältnis von 2 : 1 bis 1 : 2, insbesondere etwa äquimolar, einzusetzen. Beim Einsatz von Verbindungen der Komponente B, die eine oder mehrere Aminogruppen enthalten, werden die Verbindungen der Formel I einerseits und die Verbindungen der Komponente B andererseits bevorzugt in solchen Mengenverhältnissen eingesetzt, daß die Summe der Zahl der Carbonylgruppen der Verbindungen der Formel I und die Summe der Zahl der Aminogruppen der Verbindungen der Komponente B im Verhältnis 2 : 1 bis 1 : 2 stehen.

Zur Erlangung weiterer und intensiverer Ausfärbungen können die erfindungsgemäßen Mittel können zusätzlich Farbverstärker enthalten. Die Farbverstärker sind vorzugsweise ausgewählt aus der Gruppe bestehend aus Piperidin, Piperidin-2-carbonsäure, Piperidin-3-carbonsäure, Piperidin-4-carbonsäure, Pyridin, 2-Hydroxypyridin, 3-Hydroxypyridin, 4-Hydroxypyridin, Imidazol, 1-Methylimidazol, Histidin, Pyrrolidin, Prolin, Pyrrolidon, Pyrrolidon-5-carbonsäure, Pyrazol, 1,2,4-Triazol, Piperazidin, deren Derivate sowie deren physiologisch verträglichen Salzen.

Die voranstehend genannten Farbverstärker können in einer Menge von jeweils 0,03 bis 65, insbesondere 1 bis 40 mmol, jeweils bezogen auf 100 g des gesamten Färbemittels, eingesetzt werden.

Auf die Anwesenheit von Oxidationsmitteln, z.B. H₂O₂, kann bei der erfindungsgemäßen Verwendung der Formyl-1-methylchinolinium-Verbindungen der Formel I verzichtet werden. Es kann jedoch u.U. wünschenswert sein, den erfindungsgemäßen Mitteln zur Erzielung von Nuancen, die heller als die zu färbende keratinhaltige Faser sind, Wasserstoffperoxid oder andere Oxidationsmittel zuzusetzen. Oxidationsmittel werden in der Regel in einer Menge von 0,01 bis 6 Gew.-%, bezogen auf die Anwendungslösung, eingesetzt. Ein für menschliches Haar bevorzugtes Oxidationsmittel ist H₂O₂.

In einer bevorzugten Ausführungsform enthalten die erfindungsgemäßen Färbemittel zur weiteren Modifizierung der Farbnuancen neben den erfindungsgemäß enthaltenen Verbindungen zusätzlich übliche Oxidationshaarfarben oder direktziehende Farbstoffe. Die direktziehenden Farbstoffe können z. B. ausgewählt sein aus der Gruppe der Nitrophenylendiamine, Nitroaminophenole, Anthrachinone oder Indophenole, wie z.B. die unter den internationalen Bezeichnungen bzw. Handelsnamen HC Yellow 2, HC Yellow 4, HC Yellow 6, Basic Yellow 57, Disperse Orange 3, HC Red 3, HC Red BN, Basic Red 76, HC Blue 2, Disperse Blue 3, Basic Blue 99, HC Violet 1, Disperse Violet 1, Disperse Violet4, Disperse Black 9, Basic Brown 16, Basic Brown 17, Pikraminsäure und 2-Amino-6-chloro-4-nitrophenol bekannten Verbindungen sowie 4-Amino-2-nitrodiphenylamin-2'-carbonsäure, 6-Nitro-1,2,3,4-tetrahydrochinoxalin, 4-N-Ethyl-1,4-bis(2'-hydroxyethylamino)-2-nitrobenzol-hydrochlorid und 1-Methyl-3-nitro-4-(2'-hydroxyethyl)-aminobenzol. Die erfindungsgemäßen Mittel gemäß dieser Ausführungsform enthalten die direktziehenden Farbstoffe bevorzugt in einer Menge von 0,01 bis 20 Gew.-%, bezogen auf das gesamte Färbemittel.

Die erfindungsgemäßen Färbemittel ergeben bereits bei physiologisch verträglichen Temperaturen von unter 45°C intensive Färbungen. Sie eignen sich deshalb besonders zum Färben von menschlichen Haaren. Zur Anwendung auf dem menschlichen Haar können die Färbemittel üblicherweise in einen wasserhaltigen kosmetischen Träger eingearbeitet werden. Geeignete wasserhaltige kosmetische Träger sind z.B. Cremes, Emulsionen, Gele oder auch tensidhaltige schäumende Lösungen wie z.B. Shampoos oder andere Zubereitungen, die für die Anwendung auf den keratinhaltigen Fasern geeignet sind. Falls erforderlich ist es auch möglich, die Färbemittel in wasserfreie Träger einzuarbeiten.

Weiterhin können die erfindungsgemäßen Färbemittel alle in solchen Zubereitungen bekannten Wirk-, Zusatz- und Hilfsstoffe enthalten. In vielen Fällen enthalten die Färbemittel mindestens ein Tensid, wobei prinzipiell sowohl anionische als auch zwitterionische, ampholytische, nichtionische und kationische Tenside geeignet sind. In vielen Fällen hat es sich aber als vorteilhaft erwiesen, die Tenside aus anionischen, zwitterionischen oder nichtionischen Tensiden auszuwählen.

Als anionische Tenside eignen sich in erfindungsgemäßen Zubereitungen alle für die Verwendung am menschlichen Körper geeigneten anionischen oberflächenaktiven Stoffe. Diese sind gekennzeichnet durch eine wasserlöslich machende, anionische Gruppe wie z. B. eine Carboxylat-, Sulfat-, Sulfonat- oder Phosphat-Gruppe und eine lipophile Alkylgruppe mit etwa 10 bis 22 C-Atomen. Zusätzlich können im Molekül Glykol- oder Polyglykolether-Gruppen, Ester-, Ether- und Amidgruppen sowie Hydroxylgruppen enthalten sein. Beispiele für geeignete anionische Tenside sind, jeweils in Form der Natrium-, Kalium- und Ammonium- sowie der Mono-, Di- und Trialkanolammoniumsalze mit 2 oder 3 C-Atomen in der Alkanolgruppe,
- lineare Fettsäuren mit 10 bis 22 C-Atomen (Seifen),
- Ethercarbonsäuren der Formel R-O-(CH₂-CH₂O)ₓ -CH₂-COOH, in der R eine lineare Alkylgruppe mit 10 bis 22 C-Atomen und x = 0 oder 1 bis 16 ist,
- Acylsarcoside mit 10 bis 18 C-Atomen in der Acylgruppe,
- Acyltauride mit 10 bis 18 C-Atomen in der Acylgruppe,
- Acylisethionate mit 10 bis 18 C-Atomen in der Acylgruppe,
- Sulfobemsteinsäuremono- und -dialkylester mit 8 bis 18 C-Atomen in der Alkylgruppe und Sulfobemsteinsäuremono-alkylpolyoxyethylester mit 8 bis 18 C-Atomen in der Alkylgruppe und 1 bis 6 Oxyethylgruppen,
- lineare Alkansulfonate mit 12 bis 18 C-Atomen,
- lineare Alpha-Olefinsulfonate mit 12 bis 18 C-Atomen,
- Alpha-Sulfofettsäuremethylester von Fettsäuren mit 12 bis 18 C-Atomen,
- Alkylsulfate und Alkylpolyglykolethersulfate der Formel R-O(CH₂-CH₂O)ₓ-SO₃H, in der R eine bevorzugt lineare Alkylgruppe mit 10 bis 18 C-Atomen und x = 0 oder 1 bis 12 ist,
- Gemische oberflächenaktiver Hydroxysulfonate gemäß DE-A-37 25 030,
- sulfatierte Hydroxyalkylpolyethylen- und/oder Hydroxyalkylenpropylenglykolether gemäß DE-A-37 23 354,
- Sulfonate ungesättigter Fettsäuren mit 12 bis 24 C-Atomen und 1 bis 6 Doppelbindungen gemäß DE-A-39 26 344,
- Ester der Weinsäure und Zitronensäure mit Alkoholen, die Anlagerungsprodukte von etwa 2-15 Molekülen Ethylenoxid und/oder Propylenoxid an Fettalkohole mit 8 bis 22 C-Atomen darstellen.

Bevorzugte anionische Tenside sind Alkylsulfate, Alkylpolyglykolethersulfate und Ethercarbonsäuren mit 10 bis 18 C-Atomen in der Alkylgruppe und bis zu 12 Glykolethergruppen im Molekül sowie insbesondere Salze von gesättigten und insbesondere ungesättigten C₈-C₂₂-Carbonsäuren, wie Ölsäure, Stearinsäure, Isostearinsäure und Palmitinsäure.

Als zwitterionische Tenside werden solche oberflächenaktiven Verbindungen bezeichnet, die im Molekül mindestens eine quartäre Ammoniumgruppe und mindestens eine -COO⁽⁻⁾- oder -SO₃⁽⁻⁾-Gruppe tragen. Besonders geeignete zwitterionische Tenside sind die sogenannten Betaine wie die N-Alkyl-N,N-dimethylammonium-glycinate, beispielsweise das Kokosalkyl-dimethylammoniumglycinat, N-Acyl-aminopropyl-N,N-dimethylammoniumglycinate, beispielsweise das Kokosacylaminopropyldimethylammoniumglycinat, und 2-Alkyl-3-carboxymethyl-3-hydroxyethyl-imidazoline mit jeweils 8 bis 18 C-Atomen in der Alkyl- oder Acylgruppe sowie das Kokosacylaminoethylhydroxyethylcarboxymethylglycinat. Ein bevorzugtes zwitterionisches Tensid ist das unter der CTFA-Bezeichnung Cocamidopropyl Betaine bekannte Fettsäureamid-Derivat.

Unter ampholytischen Tensiden werden solche oberflächenaktiven Verbindungen verstanden, die außer einer C₈₋₁₈-Alkyl- oder -Acylgruppe im Molekül mindestens eine freie Aminogruppe und mindestens eine -COOH- oder -SO₃H-Gruppe enthalten und zur Ausbildung innerer Salze befähigt sind. Beispiele für geeignete ampholytische Tenside sind N-Alkylglycine, N-Alkylpropionsäuren, N-Alkylaminobuttersäuren, N-Alkyliminodipropionsäuren, N-Hydroxyethyl-N-alkylamidopropylglycine, N-Alkyltaurine, N-Alkylsarcosine, 2-Alkylaminopropionsäuren und Alkylaminoessigsäuren mit jeweils etwa 8 bis 18 C-Atomen in der Alkylgruppe. Besonders bevorzugte ampholytische Tenside sind das N-Kokosalkylaminopropionat, das Kokosacylaminoethylaminopropionat und das C₁₂₋₁₈-Acylsarcosin.

Nichtionische Tenside enthalten als hydrophile Gruppe z. B. eine Polyolgruppe, eine Polyalkylenglykolethergruppe oder eine Kombination aus Polyol- und Polyglykolethergruppe. Solche Verbindungen sind beispielsweise
- Anlagerungsprodukte von 2 bis 30 Mol Ethylenoxid und/oder 0 bis 5 Mol Propylenoxid an lineare Fettalkohole mit 8 bis 22 C-Atomen, an Fettsäuren mit 12 bis 22 C-Atomen und an Alkylphenole mit 8 bis 15 C-Atomen in der Alkylgruppe,
- C₁₂₋₂₂-Fettsäuremono- und -diester von Antagerungsprodukten von 1 bis 30 Mol Ethylenoxid an Glycerin,
- C₈₋₂₂-Alkylmono- und -oligoglycoside und deren ethoxylierte Analoga,
- Anlagerungsprodukte von 5 bis 60 Mol Ethylenoxid an Rizinusöl und gehärtetes Rizinusöl,
- Anlagerungeprodukte von Ethylenoxid an Sorbitanfettsäureester
- Anlagerungsprodukte von Ethylenoxid an Fettsäurealkanolamide.

Beispiele für die in den erfindungsgemäßen Haarbehandlungsmitteln verwendbaren kationischen Tenside sind insbesondere quartäre Ammoniumverbindungen. Bevorzugt sind Ammoniumhalogenide wie Alkyltrimethylammoniumchloride, Dialkyldimethylammoniumchloride und Trialkylmethylammoniumchloride, z. B. Cetyltrimethylammoniumchlorid, Stearyltrimethylammoniumchlorid, Distearyldimethylammoniumchlorid, Lauryldimethylammoniumchlorid, Lauryldimethylbenzylammoniumchlorid und Tricetylmethylammoniumchlorid. Weitere erfindungsgemäß verwendbare kationische Tenside stellen die quatemisierten Proteinhydrolysate dar.

Erfindungsgemäß ebenfalls geeignet sind kationische Silikonöle wie beispielsweise die im Handel erhältlichen Produkte Q2-7224 (Hersteller: Dow Coming; ein stabilisiertes Trimethylsilylamodimethicon), Dow Coming 929 Emulsion (enthaltend ein hydroxylamino-modifiziertes Silicon, das auch als Amodimethicone bezeichnet wird), SM-2059 (Hersteller: General Electric), SLM-55067 (Hersteller: Wacker) sowie Abil®-Quat 3270 und 3272 (Hersteller: Th. Goldschmidt; diquatemäre Polydimethylsiloxane, Quaternium-80).

Alkylamidoamine, insbesondere Fettsäureamidoamine wie das unter der Bezeichnung Tego Amid®S 18 erhältliche Stearylamidopropyldimethylamin, zeichnen sich neben einer guten konditionierenden Wirkung speziell durch ihre gute biologische Abbaubarkeit aus.

Ebenfalls sehr gut biologisch abbaubar sind quatemäre Esterverbindungen, sogenannte "Esterquats", wie die unter dem Warenzeichen Stepantex® vertriebenen Methylhydroxyalkyldialkoyloxyalkylammoniummethosulfate.

Ein Beispiel für ein als kationisches Tensid einsetzbares quatemäres Zuckerderivat stellt das Handelsprodukt Glucquat®100 dar, gemäß CTFA-Nomenklatur ein "Lauryl Methyl Gluceth-10 Hydroxypropyl Dimonium Chloride".

Bei den als Tenside eingesetzten Verbindungen mit Alkylgruppen kann es sich jeweils um einheitliche Substanzen handeln. Es ist jedoch in der Regel bevorzugt, bei der Herstellung dieser Stoffe von nativen pflanzlichen oder tierischen Rohstoffen auszugehen, so daß man Substanzgemische mit unterschiedlichen, vom jeweiligen Rohstoff abhängigen Alkylkettenlängen erhält.

Bei den Tensiden, die Anlagerungsprodukte von Ethylen- und/oder Propylenoxid an Fettalkohole oder Derivate dieser Anlagerungsprodukte darstellen, können sowohl Produkte mit einer "normalen" Homologenverteilung als auch solche mit einer eingeengten Homologenverteilung verwendet werden. Unter "normaler" Homologenverteilung werden dabei Mischungen von Homologen verstanden, die man bei der Umsetzung von Fettalkohol und Alkylenoxid unter Verwendung von Alkalimetallen, Alkalimetallhydroxiden oder Alkalimetallalkoholaten als Katalysatoren erhält. Eingeengte Homologenverteilungen werden dagegen erhalten, wenn beispielsweise Hydrotalcite, Erdalkalimetallsalze von Ethercarbonsäuren, Erdalkalimetalloxide, -hydroxide oder -alkoholate als Katalysatoren verwendet werden. Die Verwendung von Produkten mit eingeengter Homologenverteilung kann bevorzugt sein.

Weitere Wirk-, Hilfs- und Zusatzstoffe sind beispielsweise
- nichtionische Polymere wie beispielsweise Vinylpyrrolidon/Vinylacrylat-Copolymere, Polyvinylpyrrolidon und Vinylpyrrolidon/Vinylacetat-Copolymere und Polysiloxane,
- kationische Polymere wie quatemisierte Celluloseether, Polysiloxane mit quaternären Gruppen, Dimethyldiallylammoniumchlorid-Polymere, Acrylamid-Dimethyldiallylammoniumchlorid-Copolymere, mit Diethylsulfat quatemierte Dimethylaminoethylmethacrylat-Vinylpyrrolidon-Copolymere, Vinylpyrrolidon-Imidazoliniummethochlorid-Copolymere und quaternierter Polyvinylalkohol,
- zwitterionische und amphotere Polymere wie beispielsweise Acrylamidopropyltrimethylammoniumchlorid/Acrylat-Copolymere und Octylacrylamid/Methylmethacrylat/tert.-Butylaminoethylmethacrylat/2-Hydroxypropylmethacrylat-Copolymere,
- anionische Polymere wie beispielsweise Polyacrylsäuren, vernetzte Polyacrylsäuren, Vinylacetat/Crotonsäure-Copolymere, Vinylpyrrolidon/Vinylacrylat-Copolymere, Vinylacetat/Butylmaleat/Isobomylacrylat-Copolymere, Methylvinylether/Maleinsäureanhydrid-Copolymere und Acrylsäure/Ethylacrytat/N-tert.-Butylacrylamid-Terpolymere,
- Verdickungsmittel wie Agar-Agar, Guar-Gum, Alginate, Xanthan-Gum, Gummi arabicum, Karaya-Gummi, Johannisbrotkemmehl, Leinsamengummen, Dextrane, Cellulose-Derivate, z. B. Methylcellulose, Hydroxyalkylcellulose und Carboxymethylcellulose, Stärke-Fraktionen und Derivate wie Amylose, Amylopektin und Dextrine, Tone wie z. B. Bentonit oder vollsynthetische Hydrokolloide wie z.B. Polyvinylalkohol,
- Strukturanten wie Glucose und Maleinsäure,
- haarkonditionierende Verbindungen wie Phospholipide, beispielsweise Sojalecithin, Ei-Lecitin und Kephaline, sowie Silikonöle,
- Proteinhydrolysate, insbesondere Elastin-, Kollagen-, Keratin-, Milcheiweiß-, Sojaprotein- und Weizenproteinhydrolysate, deren Kondensationsprodukte mit Fettsäuren sowie quatemisierte Proteinhydrolysate,
- Parfümöle, Dimethylisosorbid und Cyclodextrine,
- Lösungsvermittler wie Ethanol, Isopropanol, Ethylenglykol, Propylenglykol, Glycerin und Diethylenglykol,
- Antischuppenwirkstoffe wie Piroctone Olamine und Zink Omadine,
- weitere Substanzen zur Einstellung des pH-Wertes,
- Wirkstoffe wie Panthenol, Pantothensäure, Allantoin, Pyrrolidoncarbonsäuren und deren Salze, Pflanzenextrakte und Vitamine,
- Cholesterin,
- Lichtschutzmittel,
- Konsistenzgeber wie Zuckerester, Polyolester oder Polyolalkylether,
- Fette und Wachse wie Walrat, Bienenwachs, Montanwachs, Paraffine, Fettalkohole und Fettsäureester,
- Fettsäurealkanolamide,
- Komplexbildner wie EDTA, NTA und Phosphonsäuren,
- Quell- und Penetrationsstoffe wie Glycerin, Propylenglykolmonoethylether, Carbonate, Hydrogencarbonate, Guanidine, Harnstoffe sowie primäre, sekundäre und tertiäre Phosphate, Imidazole, Tannine, Pyrrol,
- Trübungsmittel wie Latex,
- Perlglanzmittel wie Ethylenglykolmono- und -distearat,
- Treibmittel wie Propan-Butan-Gemische, N₂O, Dimethylether, CO₂ und Luft sowie
- Antioxidantien.

Die Bestandteile des wasserhaltigen Trägers werden zur Herstellung der erfindungsgemäßen Färbemittel in für diesen Zweck üblichen Mengen eingesetzt; z.B. werden Emulgiermittel in Konzentrationen von 0,5 bis 30 Gew.-% und Verdickungsmittel in Konzentrationen von 0,1 bis 25 Gew.-% des gesamten Färbemittels eingesetzt.

Für das Färbeergebnis kann es vorteilhaft sein, den Färbemitteln Ammonium- oder Metallsalze zuzugeben. Geeignete Metallsalze sind z.B. Formiate, Carbonate, Halogenide, Sulfate, Butyrate, Valeriate, Capronate, Acetate, Lactate, Glykolate, Tartrate, Citrate, Gluconate, Propionate, Phosphate und Phosphonate von Erdalkalimetallen, wie Kalium, Natrium oder Lithium, Erdalkalimetallen, wie Magnesium, Calcium, Strontium oder Barium, oder von Aluminium, Mangan, Eisen, Kobalt, Kupfer oder Zink, wobei Natriumacetat, Lithiumbromid, Calciumbromid, Calciumgluconat, Zinkchlorid, Zinksulfat, Magnesiumchlorid, Magnesiumsulfat, Ammoniumcarbonat, -chlorid und -acetat bevorzugt sind. Diese Salze sind vorzugsweise in einer Menge von 0,03 bis 65, insbesondere von 1 bis 40 mmol, bezogen auf 100 g des gesamten Färbemittels, enthalten.

Der pH-Wert der gebrauchsfertigen Färbezubereitungen liegt üblicherweise zwischen 2 und 11, vorzugsweise zwischen 5 und 9.

Zum Färben der keratinhaltigen Fasern, insbesondere zum Färben von menschlichen Haaren, werden die Färbemittel in der Regel gemeinsam mit einem wasserhaltigen, kosmetischen Träger in einer Menge von 100 g auf das Haar aufgebracht, ca. 30 Minuten dort belassen und anschließend ausgespült oder mit einem handelsüblichen Haarshampoo ausgewaschen. Die fakultativ enthaltenen Ammonium- oder Metallsalze können allein oder im Gemisch mit den weiteren Komponenten zugesetzt werden. Auch eine Vorbehandlung der Fasern mit der Salzlösung ist möglich.

Die einzelnen Komponenten der erfindungsgemäßen Mittel können entweder getrennt oder zusammen gelagert werden, entweder in einer flüssigen bis pastösen Zubereitung (wäßrig oder wasserfrei) oder als trockenes Pulver. Werden die Komponenten in einer flüssigen Zubereitung zusammen gelagert, so sollte diese zur Verminderung einer Reaktion der Komponenten weitgehend wasserfrei sein. Bei der getrennten Lagerung werden die reaktiven Komponenten erst unmittelbar vor der Anwendung miteinander innig vermischt. Bei der trockenen Lagerung wird vor der Anwendung üblicherweise eine definierte Menge warmen (50 bis 80°C) Wassers hinzugefügt und eine homogene Mischung hergestellt.

### Beispiele

### Beispiel 1:

### Herstellung von 4-Formyl-1-methylchinolinium-p-toluolsulfonat

Eine Lösung von 20,0g (128 mmol) Chinolin-4-carboxaldehyd und 35,8g p-Toluolsulfonsäuremethylester in 100 ml Toluol wurde unter Rühren zunächst 4 Std. auf 80°C erwärmt. Danach wurde die Mischung auf Siedetemperatur erhöht, worauf sich nach kurzer Zeit ein gelber Niederschlag bildete, der allmählich eine harzartige Konsistenz annahm. Anschließend wurde die Temperatur auf etwa 60 bis 70°C abgesenkt und gerührt. Es bildete sich ein kristallines Produkt. Das Reaktionsgemisch wurde noch einmal 8 Std. auf Siedetemperatur gerührt, nach dem Abkühlen wurde das Produkt abfiltriert, mit Toluol gewaschen und bei 40°C unter vermindertem Druck getrocknet. Die erhaltenen Kristalle hatten einen Schmelzpunkt von 164°C.
Ausbeute: 99,9 %
Reinheit: 100% nach 1 H-NMR.

### Beispiel 2:

### Ausfärbungen

Als Komponente I wurde eine erfindungsgemäße Formyl-1-methylchinolinium-Verbindung mit Wasser und einem Verdickungsmittel in den in Tabelle 1 wiedergegebenen Mengenanteilen vermischt [alle Angaben sind, soweit nicht anders vermerkt, in Gew.-%].

**Tabelle 1**

| | **Komponente IA** | **Komponente IB** |
|---|---|---|
| 4- Formyl-1-methylchinolinium-p-toluolsulfonat | 3,61 | - |
| 2- Formyl-1-methylchinolinium-p-toluolsulfonat | - | 7,22 |
| Natrosol® HD 250' | 2,00 | 2,00 |
| Wasser, dest., | 94,39 | 90,78 |

| | | |
|---|---|---|
| 1 Hydroxyethylcellulose (Handelsprodukt der Fa. Hercules) | | |

Als Komponente II wurde eine Cremebasis der in Tabelle 2 wiedergegebenen Zusammensetzung hergestellt [alle Angaben sind, soweit nicht anders vermerkt, in Gew.-%]

**Tabelle 2**

| | II-1 | II-2 | II-3 | II-4 | II-5 | II-6 | II-7 | II-8 |
|---|---|---|---|---|---|---|---|---|
| Texapon®NSO² | 13,00 | 13,00 | 13,00 | 13,00 | 26,00 | 26,00 | 26,00 | 26,00 |
| Talgfettalkohol | 4,25 | 4,25 | 4,25 | 4,25 | 8,50 | 8,50 | 8,50 | 8,50 |
| Lorol® techn³ | 1,0 | 1,0 | 1,0 | 1,0 | 2,00 | 2,00 | 2,00 | 2,00 |
| Ascorbinsäure | 0,1 | 0,1 | 0,1 | 0,1 | 0,1 | 0,1 9 | 0,1 | 0,1 |
| Natriumsulfit | 0,1 | 0,1 | 0,1 | 0,1 | 0,1 | 0,1 | 0,1 | 0,1 |
| N,N-Bis(2-hydroxyethyl)-p-phenylendiamin | 2,94 | - | - | - | - | - | - | - |
| p-Toluylendiaminsulfat | - | 2,21 | - | - | 4,41 | - | - | - |
| 3-Methyl-p-aminophenol | - | - | 1,23 | - | - | - | - | 2,46 |
| 4-Amino-2-aminomethyl-1-hydroxybenzol | - | - | - 2,12 | | - | - | - | - |
| 2,4,5,6-Tetraaminopyrimidinsulfat | - | - | - | - | - | 4,76 | - | - |
| 2-Methylamino-3-amino-6-methoxypyridin | - | - | - | - | - | - | 4,52 | - |
| Ammoniak, 25 %ig ad pH | 6,20 | 9,00 | 6,00 | 9,10 | 9,01 | 9,10 | 9,00 | 9,00 |
| destilliertes Wasser | ad 100 | | | | | | | |

| | | | | | | | | |
|---|---|---|---|---|---|---|---|---|
| 2 C_{12/14}-Fettalkoholpolyglycolether(2EO)sulfat (Hersteller: COGNIS) | | | | | | | | |
| 3 C₁₂₋₁₈-Fettalkohol (Hersteller: COGNIS) | | | | | | | | |

Die Komponente 1 wurde im Gewichtsverhältnis 1 : 1 mit der Komponente II verrührt. Es wurden die Komponente IA mit den Komponenten II-1 bis II-4 und die Komponente IB mit den Komponenten II-5 bis II-8 vermischt.

Die Färbecreme wurde auf ca. 5 cm lange Strähnen standardisierten, zu 90 % ergrauten, aber nicht besonders vorbehandelten Menschenhaars aufgetragen und dort 30 Minuten bei 32 °C belassen. Nach Beendigung des Färbeprozesses wurde das Haar gespült, mit einem üblichen Haarwaschmittel gewaschen und anschließend getrocknet.

Die Färbeergebnisse sind in der folgenden Tabelle 3 wiedergegeben:

**Tabelle 3**

| **Kombination** | **Ausfärbung** |
|---|---|
| I-A mit II-1 | Tiefviolett |
| I-A mit II-2 | dunkel violettbraun |
| I-A mit II-3 | Gelbrot |
| I-A mit 11-4 | Rotbraun |
| I-B mit II-5 | Schwarzmagenta |
| I-B mit II-6 | Violettbraun |
| I-B mit II-7 | Dunkelblau |
| I-B mit II-8 | Dunkelbraun |

### Beispiel 3:

### Ausfärbungen:

Die Färbemischungen wurden jeweils frisch hergestellt. Dazu wurden 50 g der in Tabelle 4 aufgeführten Cremes A bis J mit den in Tabelle 5 angegebenen Mengen an Farbstoffen versetzt, mit 25 %iger wäßriger Ammoniaklösung oder mit Weinsäure auf den in Tabelle 5 angegebenen pH-Wert gebracht und ggf. mit Wasser zu 100 g ergänzt. Die Ausfärbungen erfolgten dann analog Beispiel 2.

**Tabelle 4: Basiscremes**

| | A | B | C | D | E | F | G | H | I | J |
|---|---|---|---|---|---|---|---|---|---|---|
| Texapon® NSO | 52,00 | 52,00 | 52,00 | 52,00 | 52,00 | 52,00 | 52,00 | 52,00 | 52,00 | 52,00 |
| Talgfettalkohol | 17,00 | 17,00 | 17,00 | 17,00 | 17,00 | 17,00 | 17,00 | 17,00 | 17,00 | 17,00 |
| Lorol®, techn. | 4,00 | 4,00 | 4,00 | 4,00 | 4,00 | 4,00 | 4,00 | 4,00 | 4,00 | 4,00 |
| Ascorbinsäure | 0,10 | 0,10 | 0,10 | 0,10 | 0,10 | 0,10 | 0,10 | 0,10 | 0,10 | 0,10 |
| Natriumsulfit | 0,10 | 0,10 | 0,10 | 0,10 | 0,10 | 0,10 | 0,10 | 0,10 | 0,10 | 0,10 |
| Dow Corning ®929⁴ | - | 1,00 | - | - | - | - | - | - | - | - |
| Abil Quat® 3270⁵ | - | - | 1,00 | - | - | - | - | - | | |
| Luviskol® K30⁶ | - | - | - | 2,00 | - | - | - | - | - | - |
| Luviquat® FC 550⁷ | - | - | - | - | 2,00 | - | - | - | - | - |
| Polymer JR® 400⁸ | - | - | - | - | - | 2,00 | - | - | - | - |
| 1,2-Propand iol | - | - | - | - | - | - | 2,00 | - | - | - |
| Imidazol | - | - | - | - | - | - | - | 2,00 | - | - |
| Hydrotri tium® QS⁹ | - | - | - | - | - | - | - | - | 1,00 | - |
| Crotein M¹⁰ | - | - | - | - | - | - | - | - | - | 1,00 |
| Wasser, dest. | ad 100 | | | | | | | | | |

| | | | | | | | | | | |
|---|---|---|---|---|---|---|---|---|---|---|
| 4 modifiziertes Silicone (35 % Aktivsubstanz; INCI-Bezeichnung: Amodimethicone, Tallowtrimonium Chloride, Nonoxynol-10) (DOW CORNING) | | | | | | | | | | |
| 5 modifiziertes Polydimethylsiloxan (50 % Aktivsubstanz; INCI-Bezeichnung: Quatemium-80) (Goldschmidt) | | | | | | | | | | |
| 6 Polyvinylpyrrolidon (30 % Aktivsubstanz in Wasser; INCI-Bezeichnung: PVP) (BASF) | | | | | | | | | | |
| 7 Vinylimidazoliummethochlorid-Vinylpyrrolidon-Copolymerisat 50:50 (ca. 40 % Aktivsubstanz; INCI-Bezeichnung: Polyquatemium-16 (BASF) | | | | | | | | | | |
| 8 quatemierte Hydroxyethylcellulose (INCI-Bezeichnung: Polyquatemium-10) (UNION CARBIDE) | | | | | | | | | | |
| 9 quaterniertes Weizenproteinhydrolysat (ca. 30 % Aktivsubstanz; INCI-Bezeichnung: Steardimonium Hydroxypropyl Hydrolyzed Wheat Protein) (CRODA) | | | | | | | | | | |
| 10 Collagen-Hydrolysat (INCl-Bezeichnung: Hydrolyzed Marine Collagen) (CRODA) | | | | | | | | | | |

**Tabelle 5**

| **Nr.** | **Creme** | **Farbstoff** | **pH-Wert** | **Farbergebnis** |
|---|---|---|---|---|
| 9 | A | 2,5mmol 4-Formyl-1-methylchinolinium-p-toluolsulfonat + 2,5mmol Isatin + 5mmol 4,5-Diamino-1(2-hydroxyethyl)pyrazol | 9 | cubarot |
| 10 | D | 2,5mmol 4-Formyl-1-methylchinolinium-p-toluolsulfonat + 2,5mmol Isatin + 5mmol 1,4-Bis(4-aminophenyl)diazacycloheptan | 9 | dunkelblau |
| 11 | I | 2,5mmol 2-Formyl-1-methylchinolinium-p-toluolsulfonat + 2,5mmol Isatin + 5mmol 2,4,5,6-Tetraaminopyrimidinsulfat | 9 | granatbraun |
| 12 | B | 2,5mmol 2-Formyl-1-methylchinolinium-p-toluolsulfonat + 2,5mmol N-Allylisatin + 5mmol 2,4,5,6-Tetraaminopyrimidinsulfat | 9 | fuchsrot |
| 13 | G | 2,5mmol 2-Formyl-1-methylchinolinium-p-toluolsulfonat + 2,5mmol Isatin-5-sulfonsäure + 5mmol 4,5-Diamino-1(2-hydroxyethyl)pyrazol | 9 | rot |
| 14 | E | 2,5mmol 2-Formyl-1-methylchinolinium-p-toluolsulfonat + 2,5mmol Isatin-5-sulfonsäure + 5mmol N,N-Bis-(2-hydroxyethyl)-p-phenylendiamin | 9 | tiefviolett |
| 15 | H | 2,5mmol 2-Formyl-1-methylchinolinium-p-toluolsulfonat+ 2,5mmol 2,4-Dinitrobenzaldehyd + 5mmol 2,4,5,6-Tetraaminopyrimidinsulfat | 9 | Granatbraun |
| 16 | J | 2,5mmol 4-Formyl-1-methylchinolinium-p-toluolsulfonat + 2,5mmol 4-Formyl-1-methylpyridiniumbenzolsulfonat + 5mmol 1,4-Bis (4-arninophenyl)diazacycloheptan | 9 | dunkelviolett |
| 17 | B | 2,5mmol 4-Formyl-1-methylchinolinium-p-toluolsulfonat + 2,5mmol 4-Benzoyl-1-methylpyridiniummethansulfonat + 5mmol 2-(β-Hydroxyethyl)p-phenylendiaminsulfat | 9 | tief-violett |
| 18 | C | 2.5mmol 4-Formyl-1-methylchinolinium-p-toluotsulfonat + 2,5mmol Isatin-5-sulfonsäure + 5mmol 2-(β-Hydroxyethyl)p-phenylendiaminsulfat | 9 | dunkelviolett |
| 19 | I | 5mmol 4-Formyl-1-methylchinolinium-p-toluolsulfonat + 5mmol 1-(2-Hydroxy-5-aminobenzyl)-2-imidazolidinon-hydrochlorid | 9 | braunorange |
| 20 | D | 5mmol 4-Formyl-1-methylchinolinium-p-toluolsulfonat + 5mmol 3,5-Diamino-2-methoxytoluol | 9 | braun |
| 21 | F | 5mmol 4-Formyl-1-methylchinolinium-p-toluolsulfonat + 5mmol 4-Amino-2-((4-((5-amino-2-hydroxyphenyl)methylpiperazinyl)methyl)phenol*4HCl | 9 | rotgold |
| 22 | B | 5mmol 2-Formyl-1-methylchinolinium-p-toluolsulfonat + 5mmol 3,5-Diamino-2-methoxytoluol | 6 | dunkelbra un |
| 23 | C | 5mmol 2-Formyl-1-methylchinolinium-p-toluolsulfonat + 5mmol 4-Amino-2-((4-((5-amino-2-hydroxyphenyl)methyl)-piperazinyl)methyl)phenol*4HCl | 6 | teakholzfarbig |
| 24 | H | 5mmol 2-Formyl-1-methylchinolinium-p-toluolsulfonat + 5mmol 1-(2-Hydroxy-5-aminobenzyl)-2-imidazolidinon-hydrochlorid | 9 | rußbraun |
| 25 | G | 5mmol 2-Formyl-1-methylchinolinium-p-toluolsulfonat + 5mmol 2-(Aminoethylamino)-5-aminopyridin*3HCl | 6 | rotbraun |
| 26 | I | 5mmol 4-Formyl-1-methylchinolinium-p-toluolsulfonat + 5mmol 2-(Aminoethylamino)-5-aminopyridin*3HCl | 9 | rotbraun |
| 27 | B | 5mmol 2-Formyl-1-methylchinolinium-p-toluoisulfonat+5mmol (2((2,4-Diaminophenyl)amino)ethyl-5-amino-(2-pyridyl))amin*4HCl | 9 | graubraun |
| 28 | H | 5mmol 4-Formyl-1-methylchinolinium-p-toluolsulfonat + 5mmol (2((2,4-Diaminophenyl)amino)ethyl-5-amino-(2-pyridyl))amin*4HCl | 6 | dunkelbraun |
| 29 | D | 5mmol 2-Formyl-1-methylchinolinium-p-toluolsulfonat + 5mmol (2((4-Aminophenyl)amino)ethyl-(2-pyridyl))amin*4HCl | 6 | dunkelgrün |
| 30 | A | 5mmol 4-Formyl-1-methylchinolinium-p-toluolsulfonat + 5mmol (2((4-Aminophenyl)amino)ethyl-(2-pyridyl))amin*4HCl | 9 | petersiliengrün |
| 31 | B | 10mmol 4-Formyl-1-methylchinolinium-p-toluolsulfonat + 5mmol 2,4,5,6-Tetraaminopyrimidinsulfat + 5mmol 5-Amino-2-methylphenol | 9 | violettbraun |
| 32 | F | 10mmol 4-Formyl-1-methylchinolinium-p-toluolsulfonat + 3,3mmol 2-(β-Hydroxyethyl)p-phenylendiaminsulfat + 6,7mmol N,N-Bis-(2-hydroxyethyl)-p-phenylendiamin | 9 | schwarzblau |
| 33 | J | 10mmol 4-Formyl-1-methylchinolinium-p-toluolsulfonat + 2,5mmol 2-(β-Hydroxyethyl)p-phenylendiaminsulfat + 7,5mmol 5-Amino-2-methylphenol | 9 | violettbraun |
| 34 | C | 10mmol 4-Formyl-1-methylchinolinium-p-toluolsulfonat + 5mmol 3-Methyl-p-aminophenol + 5mmol 2,4-Diaminophenoxyethanol | 9 | dunkel rotbraun |
| 35 | G | 5mmol 4-Formyl-1-methylchinolinium-p-toluolsulfonat + 5mmol 1-Methoxy-2-amino-4-β-hydroxyethylaminobenzen | 6 | dunkelblond |
| 36 | I | 5mmol 4-Formyl-1-methylchinolinium-p-toluolsulfonat + 5mmol 1-Methoxy-2-amino-4-β-hydroxyethylaminobenzen | 9 | braun |
| 37 | E | 5mmol 4-Formyl-1-methylchinolinium-p-toluolsulfonat + 5mmol 5-Aminosalicylsäure | 9 | rotgold |
| 38 | D | 5mmol 4-Formyl-1-methylchinolinium-p-toluolsulfonat + 5mmol 3,5-Diamino-2,6-dimethoxypyridin | 6 | blaugrau |
| 39 | A | 5mmol 4-Formyl-1-methylchinolinium-p-toluolsulfonat + 5mmol 5-(2-Hydroxyethylamino)-2-methylphenol | 6 | grauorange |
| 40 | H | 5mmol 4-Formyl-1-methylchinolium-p-toluolsulfonat + 5mmol 5-(2-Hydroxyethylamino)-2-methylphenol | 9 | grauorange |
| 41 | F | 5mmol 4-Formyl-1-methylchinolinium-p-toluolsulfonat + 5mmol 2-(β-Hydroxyethyl)p-phenylendiaminsulfat | 9 | schwarzviolett |
| 42 | I | 5mmol 4-Formyl-1-methylchinolinium-p-toluoisulfonat + 5mmol 2-Methylamino-3-amino-6-methoxypyridin | 9 | tiefviolett |
| 43 | G | 5mmol 4-Formyl-1-methylchinolinium-p-toluolsulfonat + 5mmol 1,10-Bis-(2,5-diaminophenyl)-1,4,7,10-tetraoxadecan*4HCl | 9 | dunkelmagenta |
| 44 | B | 5mmol 4-Formyl-1-methylchinolinium-p-toluolsulfonat + 5mmol 3,4-Diaminobenzoesäure | 9 | graumagenta |
| 45 | I | 5mmol 4-Formyl-1-methylchinolinium-p-toluolsulfonat + 5mmol 1,4-Bis(4-aminophenyl)diazacycloheptan | 6 | graublau |
| 46 | A | 5mmol 4-formyl-1-methylchinolinium-p-toluolsulfonat + 5mmol 1,4-Bis(4-aminophenyl)diazacycloheptan | 9 | tiefblau |
| 47 | C | 5mmol 4-Formyl-1-methylchinolinium-p-toluolsulfonat + 5mmol 4,5-Diamino- (2-hydroxyethyl)pyrazol | 6 | granatrot |
| 48 | H | 5mmol 4-Formyl-1-methylchinolinium-p-toluolsulfonat + 5mmol Bis(2-hydroxy-5-aminophenyl)-methan*2HCl | 9 | gelbbraun |
| 49 | G | 5mmol 4-Formyl-1-methylchinolinium-p-toluolsulfonat + 5mmol 5-Methyl-2-aminophenol | 6 | orange |
| 50 | B | 5mmol 4-Fonnyl-1-methylchinolinium-p-toluolsulfonat + 5mmol 5-Methyl-2-aminophenol | 9 | aprikosengelb |
| 51 | J | 5mmol 4-Fonnyl-1-methylchinolinium-p-toluoisulfonat + 5mmol 4.5-Diamino- (2-hydroxyethyl)pyrazol | 9 | violettbra un |
| 52 | G | 5mmol 4-Formyl-1-methylchinolinium-p-toluolsulfonat + 5mmol 2.4-Dichlor-m-aminophenol | 9 | graurot |
| 53 | I | 5mmol 4-Formyl-1-methylchinolinium-p-toluolsulfonat + 5mmol 2,6-Diaminopyridin | 9 | aprikosengelb |
| 54 | D | 5mmol 4-Formyl-1-methylchinolinium-p-toluolsulfonat + 5mmol 2,3-Diaminopyridin | 9 | violettbraun |
| 55 | E | 5mmol 4-Formyl-1-methylchinolinium-p-toluoisulfonat + 5mmol 5-Amino-2-methylphenol | 9 | orange |
| 56 | J | 5mmol 4-Formyl-1-methylchinolinium-p-toluolsulfonat + 5mmol 2-Amino-3-hydroxypyridin | 9 | braunorange |
| 57 | A | 5mmol 4-Formyl-4-methylchinolinium-p-toluolsulfonat + 5mmol 2,6-Dihydroxy-3,4-dimethylpyridin | 9 | graurot |
| 58 | B | 5mmol 4-Formyl-1-methylchinolinium-p-toluolsulfonat + 5mmol 1,3-Bis(2,4-diaminophenoxy)propan*4HCl | 9 | somali |
| 59 | D | 10mmol 4-Formyl-1-methylchinolinium-p-toluolsulfonat + 10mmol 2,4,5,6-Tetraaminopyrimidinsulfat | 9 | violettbraun |
| 60 | F | 10mmol 4-Formyl-1-methylchinolinium-p-toluolsulfonat + 10mmol 4-Hydroxy-2,5,6,triaminopyrimidinsulfat | 9 | violettbraun |
| 61 | I | 10mmol 2-Formyl-1-methylchinolinium-p-toluolsulfonat + 10mmol 5-Aminosalicylsäure | 6 | braun |
| 62 | C | 10mmol 2-Formyl-1-methylchinotinium-p-toluolsulfonat + 10mmol 5-Aminosalicylsäure | 9 | cognacbraun |
| 63 | G | 10mmol 2-Formyl-1-methylchinolinium-p-toluolsulfonat + 10mmol 3,5-Diamino-2,6-dimethoxypyridin | 6 | violettschwarz |
| 64 | J | 10mmol 2-Formyl-1-methylchinolinium-p-toluolsulfonat + 10mmol 3,5-Diamino-2,6-dimethoxypyridin | 9 | teakholzfarbig |
| 65 | F | 10mmol 2-Formyl-1-methylchinolinium-p-toluolsulfonat + 10mmol 2,6-Dihydroxy-3,4-dimethylpyridin | 6 | oliv |
| 66 | E | 10mmol 2-Formyl-1-methylchinolinium-p-toluolsulfonat + 10mmol 2,6-Dihydroxy-3,4-dimethylpyridin | 9 | graubraun |
| 67 | D | 10mmol 2-Formyl-1-methylchinolinium-p-toluolsulfonat + 10mmol 5-(2-Hydroxyethylamino)-2-methylphenol | 9 | topasgelb |
| 68 | A | 10mmol 2-Formyl-1-methylchinolinium-p-toluolsulfonat + 10mmol 1-Methoxy-2-amino-4(β-hydroxyethylamino)benzen | 9 | braun |
| 69 | H | 5mmol 2-Fonnyl-1-methylchinolinium-p-toluolsulfonat + 5mmol Bis(2-hydroxy-5-aminophenyl)-methan*2HCl | 9 | graugrün |
| 70 | C | 5mmol 2-Formyl-1-methylchinolinium-p-toluolsulfonat + 5mmol 1,4-Bis(4-aminophenyl)diazacycloheptan | 6 | tiefblau |
| 71 | J | 5mmol 2-Formyl-1-methylchinolinium-p-toluolsulfonat + 5mmol 1,4-Bis(4-aminophenyl)diazacycloheptan | 9 | schwarzblau |
| 72 | G | 5mmol 2-Formyl-1-methylchinolinium-p-toluolsulfonat + 5mmol 5-Methyl-2-aminophenol | 9 | graugelb |
| 73 | E | 5mmol 2-Formyl-1-methylchinolinium-p-toluoisulfonat + 5mmol 4,5-Diamino-1(2-hydroxyethyl)pyrazol | 6 | dunkelrubin |
| 74 | C | 5mmol 2-Formyl-1-methylchinolinium-p-toluolsulfonat + 5mmol 4,5-Diamino-1(2-hydroxyethyl)pyrazol | 9 | violettbraun |
| 75 | I | 5mmol 2-Formyl-1-methylchinolinium-p-toluolsulfonat + 5mmol 2,4-Dichlor-m-aminophenol | 9 | olivgrau |
| 76 | H | 5mmol 2-Formyl-1-methylchinolinium-p-toluolsulfonat + 5mmol 2,6-Diaminopyridin | 9 | braungrau |
| 77 | E | 5mmol 2-Formyl-1-methylchinolinium-p-toluolsulfonat + 5mmol 2,3-Diaminopyridin | 9 | violettbraun |
| 78 | B | 5mmol 2-Formyl-1-methylchinolinium-p-toluolsulfonat + 5mmol 5-Amino-2-methylphenol | 9 | rußbraun |
| 79 | F | 5mmol 2-Formyl-1-methylchinolinium-p-toluolsulfonat + 5mmol 2-Amino-3-hydroxypyridin | 9 | mattviolett |
| 80 | D | 5mmol 2-Formyl-1-methylchinolinium-p-toluolsulfonat + 5mmol 2,6-Dihydroxy-3,4-dimethylpyridin | 9 | mattviolett |
| 81 | C | 5mmol 2-Formyl-1-methylchinolinium-p-toluolsulfonat + 5mmol 4-Amino-2-aminomethyl-1-hydroxybenzol*2HCl | 6 | dunkelbraun |
| 82 | J | 5mmol 2-Formyl-1-methylchinolinium-p-toluolsulfonat + 5mmol 4-Amino-2-aminomethyl-1-hydroxybenzol*2HCl | 9 | braun |
| 83 | G | 5mmol 2-Formyl-1-methylchinolinium-p-toluolsulfonat + 5mmol 2,4-Diaminophenoxyethanol | 9 | dunkelbraun |
| 84 | E | 5mmol 2-Formyl-1-methylchinolinium-p-toluolsulfonat + 5mmol 1,3-Bis(2,4-diaminophenoxy)propan*4HCl | 9 | dunkelviolett |
| 85 | B | 5mmol 2-Formyl-1-methylchinolinium-p-toluolsulfonat + 5mmol 1,10-Bis(2,5-diaminophenyl)1,4,7,10-tetraoxadecan*4HCl | 9 | dunkelviolett |
| 86 | H | 5mmol 2-Formyl-1-methylchinolinium-p-toluolsulfonat + 5mmol N,N-Bis-(2-hydroxyethyl)-p-phenylendiamin | 9 | dunkelviolett |
| 87 | D | 5mmol 2-Formyl-1-methylchinolinium-p-toluolsulfonat + 5mmol 4-Hydroxy-2,5,6-triaminopyrimidinsulfat | 9 | rotbraun |

### Herstellung der Farbcremes aus Tabelle 5

Die Färbemischungen werden jeweils frisch hergestellt. Dazu werden 50g der Cremes A-J mit den in Tab.5 angegebenen Mengen an Farbstoffen versetzt, mit 25%igem Ammoniak oder mit Weinsäure auf den in Tab.5 genannten pH-Wert gebracht und ggf. mit Wasser ad 100 ergänzt.

## Patentansprüche

1. Verwendung von Formyl-1-methylchinolinium-Verbindungen mit der folgenden Formel I, 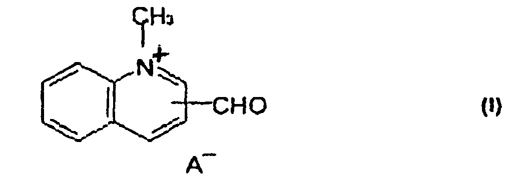 worin die Formylgruppe CHO in 2- oder 4-Position gebunden ist und A⁻ steht für ein p-Toluolsulfonat-ion, in Kombination mit mindestens einer Verbindung mit primärer oder sekundärer Aminogruppe oder Hydroxygruppe, ausgewählt aus primären oder sekundären aromatischen Aminen ausgewählt aus der Gruppe, bestehend aus N-(2-Hydroxyethyl)-N-ethyl-. N-(2-Methoxyethyl-), 2,3-, 2.4-, 2.5-Dichlor-p-phertylendiamin, 2-Chlor-p-phenylendiamin. N, N-Bis-(2-hydroxyethyt)-p-phonylendiamin. 2.5-Dihydroxy-4-morpholinoanilin-dihydrobromid, 2-, 3-, 4-Aminophenol o-, m-, p-Phenylendiamin. 2,4-Diaminophenoxyethanot, 2-(2.5-Diaminophenyl)-ethanol, 2,5-Diaminotoluol, -phenol, -phenethol, 4-Methylamino-, 3-Amino-4-(2'-hydroxyethyloxy)-, 3,4-Methylendiamino-, 3,4-Methylendioxyanilin, 3-Amino-2,4-dichlor-, 4-Methylamino-, 2-Methyl-5-amino-,3-Methyl-4-amino-, 2-Methyl-5-(2-hydroxyethylamino)-, 2-Methyl-5-amino-4-chlor-, 6-Methyl-3-amino-2-chlor-, 5-(2-Hydroxyethylamino)-4-methoxy-2-methyl-, 4-Amino-2-aminomethyl-phenol, Z-Hydroxymethyl-4-aminophenol, 2-Diethylaminomethyl-4-aminophenol. 2-Dimethylaminomelhyl-4-aminophenol, 2,6-Dichlor-4-aminophenol, 1,3-Diamino-2,4-dimethoxybenzol, 2-, 3-, 4-Aminobenzoesäure. -phenylessigsäure, 2,3-, 2,4-, 2,5-, 3,4-, 3,5-Diaminobenzoesäure. 4-, 5-Aminosalicylsäure, 3-Amino-4-hydroxy-, 4-Amino-3-hydroxybenzoesäure. 2-, 3-, 4-Aminobenzolsuffonsäure, 3-Amino-4-hydroxybenzalsulfonsäure, 4-Amino-3-hydroxynaphthalin-sulfonsäure, 6-Amino-7-hydroxynaphthalin-2-sulfonsäure, 7-Amino-4-hydroxynaphthalin-2-sulfonsäure, 4-Amino-5-hydroxynaphthalin-2.7-disulfonsäure, 3-Amino-2-naphthoesäure, 3-Aminophthalsäure, 5-Aminoisophthalsäure, 1,3,5-, 1,2,4-Triaminobenzol, 1,2,4,5-Tetraaminobenzol-tetrahydrochlorid, 2,4,5-Triaminophenol-trihydrochlorid, Pentaaminobenzol-pentahydrochlorid, Hexaaminobenzol-hexahydrochlorid, 2,4,6-Triaminoresorcin-trihydrochlorid, 4,5-Diaminobrenzcatechinsulfat, 4,6-Diaminopyrogallol-dihydrochlorid, 3,5-Diamino-4-hydroxybrenzcatechin-sulfat, 1,3-Dimethyl-2,5-diaminobenzol, 4-Amino-1-hydroxy-2-(2'-hydroxyethylaminomethyl)-benzol, 1-Hydroxy-2-amino-5-methyl-benzol, 1-Hydroxy-2-amino-6-methyl-benzol, 5-Amino-4-fluor-2-methylphenol, 2,4-Diamino-5-fluortoluol, 2,4-Diamino-5-(2'-hydroxyethoxy)-toluol. 2,4-Diamino-5-methylphenetol, 3,5-Diamino-2-methoxy-1-methylbenzol, 2-Amino-4-(2'-hydroxyethylamino)-anisol, 2,6-Bis-(2'-hydroxyethyl)-1-methylbenzol, N,N'-Bis(4-aminophenyl)-1,4-diazacycloheptan, 1-(2-Hydroxy-5-aminobenzyl)-2-imidazolidinon, 4-Amino-2-((4-[(5-amino-2-hydroxyphenyl)methyl]-piperazinyl)methyl)phenol, 4-Nitroanilin, 4-Nitro-1,3-phenylendiamin, 4-Amino-2-nitrodiphenylamin-2'-carbonsäure, 1-Amino-5-chlor-4-(2-hydroyethylamino)-2-nitrobenzol, aromatische Aniline bzw. Phenole mit einem weiteren aromatischen Rest wie 4,4'-Diaminostilben-dihydrochlorid. 4,4'-Diaminostilben-2,2'-disulfonsäure, Na-Salz, 4,4'-Diaminodiphenylmethan, -sulfid, -sulfoxid -amin. 4,4'-Diaminodiphenylamin-2-sulfonsäure, 4,4'-Diaminobenzophenon, -diphenylether, 3,3',4,4'-Tetraaminodiphenyl-tetrahydrochlorid, 3,3',4,4'-Tetraamino-benzophenon, 1,3-Bis-(2,4-diaminophenoxy)-propan-tetrahydrochlorid, 1,8-Bis-(2,5-diaminophenoxy)-3,6-dioxaoctan-tetrahydrochlorid, 1,3-Bis-(4-aminophenylamino)-propan, -2-propanol, 1,3-Bis-[N-(4-aminophenyl)-2-hydroxy-ethylamino]-2-propanol, N,N-Bis-[2-(4-aminophenoxy)-ethyl]methylamin-trihydrochlorid, Bis-(2-hydroxy-5-aminophenyl)-methan,
stickstoffhaltigen heterocyclischen Verbindungen, ausgewählt aus der Gruppe, bestehend aus
2-, 3-, 4-Amino-, 2-Amino-3-hydroxy-, 2,6-Diamino-, 2,5-Diamino-, 2,3-Diamino-, 2-Dimethylamino-5-amino-, 3-Amino-2-methylamino-6-methoxy-, 2,3-Diamino-6-methoxy-, 3,5-Diamina-2,6-dimethoxy-, 2,4,5-Triamino-, 2,6-Dihydroxy-3,4-dimethylpyridin, 4,5,6-Triamino-, 2-Hydroxy-4,5,6-triamino-, 4-Hydroxy-2,5,6-triamino-, 2,4,5.6-Tetraamino-, 2-Methylamino-4,5.6-triamino-, 2,4-, 4,5-Diamino-. 2-Amino-4-methoxy-6-methyl-pyrimidin, 2,3,4- Trimethylpyrrol. 2,4-Dimethyl-3-ethyl-pyrrot, 3,5-Diaminopyrazol, -1,2,4-triazol. 3-Amino-, 3-Aminc-5-hydroxypyrazol, 2-,3-, 8-Aminochinolin, 4-Amino-chinaldin. 2-, 6-Aminonicotinsäure. 5-Aminoisochinolin. 5-. 6-Aminoindazol, 5-, 7-Amino-benzimidazol, - benzothiazol, 2,5-Dihydroxy-4-morpholinoanilin. 1-Phenyl-3-methyl-pyratolon-5, 4,5-Diamino-1-(2-hydroxyethyl)-pyrazol, 2-(Aminoethylamino)-5-aminopyridin. (2((2,4-Diaminophenyl)amino)ethyl-5-amino-(2-pyridyl))amin, (2((4-Aminophenyl)amino)ethyl-5-amino-(2-pyridyl))amin sowie indol- und Indolinderivaten wie 4-, 5-, 6-, 7-Aminoindol, 4-, 5-, 6-, 7-Hydroxyindol 5.6-Dihydroxyindol, 5,6-Dihydroxyindolin und 4-Hydroxyindolin sowie jeweils aus den mit vorzugsweise anorganischen Säuren gebildeten physiologisch verträglichen Salzen dieser Verbindungen,
und aromatischen Hydroxyverbindungen, und/oder mindestens einer CH-aktiven Verbindung zum Färben von keratinhaltigen Fasern.

2. Mittel zum Färben von keratinhaltigen Fasern, insbesondere menschlichen Haaren, das Formyl-1-methylchinolinium-Verbindungen mit der folgenden Formel I, 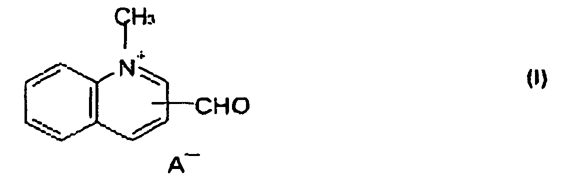 worin die Formylgruppe CHO in 2- oder 4-Position gebunden ist und A⁻ steht für ein p-Toluolsulfonat-Ion,
in Kombination mit mindestens einer Verbindung mit primärer oder Sekundarer Aminogruppe oder Hydroxygruppe, ausgewählt aus primären oder sekundären aromatischen Aminen ausgewählt aus der Gruppe, bestehend aus
N-(2-Hydroxyethy)-N-ethyl-, N-(2-Methoxyethyl-), 2,3-, 2,4-, 2,5-Dichlor-p-phenylendiamin, 2-Chlor-p-phenylendiamin, N,N-Bis-(2-hydroxyethy)-p-phenylendiamin, 2,5-Dihydroxy-4-morpholinoanilin-dihydrobromid, 2-, 3-, 4-Aminophenol, o-, m-, p-Phenylendiamin, 2,4-Diaminophenoxyethanol, 2-(2,5-Diaminophenyl)-ethanol, 2,5-Diaminotoluol, -phenol, -phenethol, 4-Methylamina-, 3-Amino-4-(2'-hydroxyethyloxy)-, 3,4-Methylendiamino-, 3,4-Methylendioxyanilin, 3-Amino-2,4-dichlor-, 4-Methylamino-, 2-Methyl-5-amino-, 3-Methyl-4-amino-, 2-Methyl-5-(2-hydroxyethylamino)-, 2-Methyl-5-amino-4-chlor-. 6-Methyl-3-amino-2-chlor-. 5-(2-Hydroxyethylamino)-4-methoxy-2-methyl-, 4-Amino-2-aminomethyl-phenol. 2-Hydroxymethyl-4-aminophenot, 2-Diethylaminomethyl-4-aminophenol. 2-Dimethylaminomethyl-4-aminophenol. 2.6-Dichlor-4-aminopheriol, 1,3-Diamino-2.4-dimethoxybenzol, 2-, 3-. 4-Aminobenzoesäure, -phenylessigsäure, 2,3-, 2.4-, 2,5-, 3.4-, 3.5-Diaminobenzoesäure. 4-, 5-Aminosalicylsäure, 3-Amino-4-hydroxy-, 4-Amino-3-hydroxybenzoesäure, 2-, 3-, 4-Aminobenzolsulfonsäure, 3-Amino-4-hydroxybenzolsulfonsäure. 4-Amino-3-hydroxynaphthalin-sulfonsäure, 6-Amino-7-hydroxynaphthalin-2-sulfansäure, 7-Amino-4-hydroxynaphthalin-2-sulfonsäure, 4-Amino-5-hydroxynaphthalin-2,7-disulfonsäure, 3-Amino-2-naphthoesäure, 3-Aminophthalsäure, 5-Aminoisophthalsäure, 1,3,5-, 1,2,4-Triaminobenzol, 1,2,4,5-Tetraaminobenzol-tetrahydrochlorid, 2,4,5-Triaminophenol-trihydrochlorid, Pentaaminobenzol-pentahydrochlorid, Hexaaminobenzol-hexahydrochlorid, 2,4,6-Triaminoresorcin-trihydrochlorid, 4,5-Diaminobrenzcatechinsulfat, 4,6-Diaminopyrogallol-dihydrochlorid, 3,5-Diamino-4-hydroxybrenzcatechin-sulfat, 1,3-Dimethyl-2,5-diaminobenzol, 4-Amino-1-hydroxy-2-(2'-hydroxyethylaminomethyl)-benzol, 1-Hydroxy-2-amino-5-methyl-benzol, 1-Hydroxy-2-amino-6-methyl-benzol, 5-Amino-4-fluor-2-methylphenol, 2.4-Diamino-5-fluortoluol, 2,4-Diamino-5-(2'-hydroxyethoxy)-toluol, 2,4-Diamino-5-methylphenetol, 3,5-Diamino-2-methoxy-1-methylbenzol, 2-Amino-4-(2'-hydroxyethylamino)-anisol, 2,6-Bis-(2'-hydroxyethyl)-1-methylbenzol, N,N'-Bis(4-aminophenyl)-1,4-diazacycloheptan, 1-(2-Hydroxy-5-aminobenzyl)-2-imidazolidinon, 4-Amino-2-((4-[(5-amino-2-hydroxyphenyl)methyl]-piperazinyl)methyl)phenol, 4-Nitroanilin, 4-Nitro-1,3-phenylendiamin, 4-Amino-2-nitrodiphenylamin-2'-carbonsäure, 1-Amino-5-chlor-4-(2-hydroyethylamino)-2-nitrobenzol, aromatische Aniline bzw. Phenole mit einem weiteren aromatischen Rest wie 4,4'-Diamiriastilben-dihydrochlorid. 4,4'-Diaminostilben-2,2'-disulfonsaure, Na-Salz. 4,4'-Diaminodiphenylmethan, -sutfid, -sulfoxid. -amin, 4,4'-Diaminodiphenylamin-2-sulfonsäure, 4,4'-Diaminobenzophenon. -diphenylether, 3,3',4.4'-Tetraanlinodiphenyl-tetrahydrochlorid. 3,3',4,4'-Tetraamino-benzophenon, 1,3-Bis-(2.4-diaminophenoxy)-propan-tetrahydrochlorid, 1,8-Bis-(2.5-diaminophenoxy)-3,6-dioxaoctari-tetrahydrochlorid. 1,3-Bis-(4-aminophenylamino)-propan, -2-propanol, 1,3-Bis-[N-(4-aminophenyl)-2-hydroxy-ethylaminvj-2-propanot, N,N-Bis-[2-(4-aminophenoxy)-ethyl]methylamin-trihydrochlorid, Bis-(2-hydroxy-5-aminophenyl)-methan.
stickstoffhaltigen heterocyclischen Verbindungen, ausgewählt aus der Gruppe, bestehend aus
2-, 3. 4-Amino-. 2-Amino-3-hydroxy-, 2.6-Diamino-, 2.5-Ofamino-, 2.3-Diamino-, 2-Dimethylamino-5-amino-, 3-Amino-2-methylamino-6-methoxy-, 2,3-Diamino-6-methoxy-, 3,5-Diamino-2,6-dimethoxy-, 2.4,5-Triamino-, 2,6-Dihydroxy-3,4-dimethylpyridin, 4,5,6-Triamino-, 2-Hydroxy-4,5,6-triamino-, 4-Hydroxy-2,5,6-triamino-, 2,4,5,6-Tetraamino-, 2-Methylamino-4,5,6-triamino-, 2,4-, 4,5-Diamino-, 2-Amino-4-methoxy-6-methyl-pyrimidin, 2,3,4-Trimethylpyrrol, 2,4-Dimethyl-3-ethyl-pyrrol, 3,5-Diaminopyrazol, -1,2,4-triazol, 3-Amino-, 3-Amino-5-hydroxypyrazol, 2-,3-, 8-Aminochinolin, 4-Amino-chinaldin, 2-, 6-Aminonicotinsäure, 5-Aminoisochinolin, 5-, 6-Aminoindazol, 5-, 7-Amino-benzimidazol, - benzothiazol, 2,5-Dihydroxy-4-morpholinoanilin, 1-Phenyl-3-methyl-pyrazolon-5, 4,5-Diamino-1-(2-hydroxyethyl)-pyrazol, 2-(Aminoethylamino)-5-aminopyridin, (2((2.4-Diaminophenyl)amino)ethyl-5-amino-(2-pyridyl))amin. (2((4-Aminophenyl)amino)ethyl-5-amino-(2-pyridyl))amin sowie Indol- und Indolinderivaten, wie 4-, 5-, 6-, 7-Aminoindol, 4-, 5-, 6-, 7-Hydroxyindol, 5,6-D:hydroxyindol, 5,6-Dihydroxyindolin und 4-Hydroxyindolin, sowie jeweils aus den mit vorzugsweise anorganischen Säuren gebildeten physiologisch verträglichen Salzen dieser Verbindungen,
und aromatischen Hydroxyverbindungen, und/oder mindestens einer CH-aktiven Verbindung enthält.

3. Mittel nach Anspruch 2, **dadurch gekennzeichnet, dass** die Verbindungen gemäß Formel (I) in Form ihrer Hydrate eingesetzt werden.

4. Mittel nach einem der Ansprüche 2 oder 3, **dadurch gekennzeichnet, daß** die Verbindungen mit der Formel I in einer Menge von jeweils 0.03 bis 65, insbesondere 1 bis 40 mmol, jeweils bezogen auf 100 g des gesamten Färbemittels, enthalten sind.

5. Mittel nach Anspruch 2. **dadurch gekennzeichnet, daß** die aromatischen Hydroxyverbindungen ausgewählt werden aus der Gruppe bestehend aus 2-, 4-, 5-Methylresorcin 2,5-Dimethylresorcin. Resorcin. 3-Methoxyphenol, Brenzkatechin Hydrochinon, Pyrogallol, Phloroglucin, Hydroxyhydrochinon, 2-. 3-, 4-Methoxy- 3-Dimethylamino-, 2-(2-Hydroxyethyl)-, 3,4-Methylendioxyphenol, 2,4-, 3,4-Dihydroxybenzoesäure, -phenylessigsäure, Gallussäure, 2,4,6-Trihydroxybenzoesäure, -acetophenon, 2-. 4-Chlorresorcin, 1-Naphthol, 1,5-, 2,3-, 2,7-Dihydroxynaphthalin, 6-Dimethylamino-4-hydroxy-2-naphthalinsulfonsäure, 3,6-Dihydroxy-2,7-naphthalinsulfonsaure.

6. Mittel nach einem der Ansprüche 2 oder 5, **dadurch gekennzeichnet, daß** die CH-aktiven Verbindungen ausgewählt werden aus der Gruppe, bestehend aus 1,2,3,3-Tetramethyl-3H-indoliumiodid. 1,2,3,3-Tetramethyl-3H-indolium-p-toluotsulfonat, 1,2,3,3-Tetramethyl-3H-indolium-methansulfonat. Fischersche Base (1,3,3-Trimethyl-2-methylenindolin), 2,3-Dimethyl-benzothiazoliumiodid, 2,3-Dimethyl-benzothiazolium-p-tatuolsulfonat, 1,2-Dimethyl-naphtho[1,2-d]thiazolium-p-toluolsulfonat, 1-Ethyl-2-methyl-naphtha[1,2-d]thiazolium-p-toluolsulfonat, Rhodanin, Rhodanin-3-essigsaure. 1-Ethyl-2-chinaldinium-iodid. 1,4-Dimethylchinolinium-iodid, 1-Methyl-2-chinaldinium-iodid. Barbitursäure. Thiobarbitursäure. 1,3-Dimethylthiobarbitursäure, 1,3-Diethylthiobarbitursäure. Diethylthiobarbitursäure, Oxindol, 3-Indoxylar-etat, Cumaranon und 1-Methyl-3-phenyl-2-pyrazolinon.

7. Mittel nach Anspruch 2, **dadurch gekennzeichnet, daß** die weitere Verbindung ausgewählt ist aus der Gruppe bestehend aus N,N-Bis(2-hydroxyethyl)p-phenylendiamin, 2,5-Diaminotoluol, 2,(2,5-Diaminphenyl)-ethanol, 2-Aminophenol, 3-Aminophenol, 4-Aminophenol, 2-Aminomethyl-4-aminophenol, 2-(Diethylaminomethyl)-4-aminophenol, 1-Hydroxy-2-amino-5-methyl-benzol, 4-Amino-3-methylphenol, 2,4-Diaminophenoxyethanol, 3-Amino-2,4-dichlorphenol, 2-Methyl-5-aminophenol, 2-Methyl-5-(2-hydroxyethylamino)-phenol, 6-Methyl-3-amino-2-chlorophenol, 2-Methyl-5-amino-4-chlorphenol, 3,4-Methylendioxyanilin, 3,4-Diaminobenzoesäure, 3,5-Diamino-2-methoxy-1-methylbenzol, 2-Amino-4-(2'-hydroxyethylamino)-anisol, 2,6-Bis-(2'-hydroxyethyl)-1-methylbenzol, 1,3-Bis-(2,4-diaminophenoxy)-propan, 4,5-Diamino-1-(2-hydroxyethyl)pyrazol, N,N'-Bis(4-aminophenyl)-1.4-diazacycloheptan. Bis-(2-hydroxy-5-aminophenyl)-methan, 1.8-Bis-(2,5-diaminophenoxy)-3,6-dioxaoctan, 4,4'-Diaminodiphenylamin, 2-Amino-3-hydroxypyridin. 3-Amino-2-methylamino-6-methoxypyridin, 2,6-Dihydroxy-3,4-dimethylpyridin, 3,5-Diamino-2,6-dimethoxypyridin 5,6-Dihydroxyindol, 5,6-Dihydroxyindolin. 2,4,5,6-Tetraaminopyrimidin, 2-Hydroxy-4,5,6-triamino-pyrimidin, 4-Hydroxy-2,5,6-triamino-pyrimidin sowie jeweils aus den vorzugsweise mit anorganischen Säuren gebildeten physiologisch verträglichen Salzen dieser Verbindungen.

8. Mittel nach einem der Ansprüche 2 bis 7, **dadurch gekennzeichnet, daß** es Farbverstärker ausgewählt aus der Gruppe bestehend aus Piperidin, Piperidin-2-carbonsäure, Piperidin-3-carbonsäure, Piperidin-4-carbonsäure, Pyridin, 2-Hydroxypyridin, 3-Hydroxypyridin, 4-Hydroxypyridin, Imidazol, 1-Methylimidazol, Histidin, Pyrrolidin, Prolin, Pyrrolidon, Pyrrolidon-5-carbonsaure, Pyrazol, 1,2,4-Triazol. Piperazidin oder deren beliebigen Gemischen enthält.

9. Mittel nach einem der Ansprüche 2 bis 8, **dadurch gekennzeichnet, daß** es direkt ziehende Farbstoffe aus der Gruppe der Nitrophenylendiamine, Nitroaminophenole, Anthrachinone oder Indophenole vorzugsweise in einer Menge von 0,01 bis 20 Gew.-%, bezogen auf das gesamte Färbemittel, enthält.

10. Mittel nach einem der Ansprüche 2 bis 9. **dadurch gekennzeichnet, daß** Ammonium- oder Metallsalze ausgewählt aus der Gruppe der Formiate. Carbonate. Halogenide. Sulfate, Butyrate, valeriate, Capronate, Acetate, Lactate, Glykolate, Tartrate, Citrate. Gluconate, Propionate. Phosphate und Phosphonate von Alkalimetallen, wie Kalium, Natrium oder Lithium. Erdalkalimetallen, wie Magnesium. Calcium. Strontium oder Barium, oder von Aluminium, Mangan, Eisen, Kobalt, Kupfer oder Zink, zugegeben werden.

11. Mittel nach einem der Ansprüche 2 bis 10, **dadurch gekennzeichnet, daß** Oxidationsmittel in einer Menge von 0,01 bis 6 Gew.-%, bezogen auf die Anwendungslösung, eingesetzt werden.

12. Mittel nach einem der Ansprüche 2 bis 11, **dadurch gekennzeichnet, daß** als Oxidationsmittel H₂O₂ eingesetzt wird.

13. Mittel nach einem der Ansprüche 2 bis 12, **dadurch gekennzeichnet, daß** anionische, zwitterionische und/oder nichtionische Tenside eingesetzt werden.

14. Verfahren zum Färben von keratinhaltigen Fasern, worin ein Färbemittel, nach einem der Ansprüche 2 bis 13 zum Färben von keratinhaltigen Fasern sowie übliche kosmetische inhaltsstoffe, auf die keratinhaltigen Fasern aufgebracht, einige Zeit, üblicherweise ca. 30 Minuten, auf der Faser belassen und anschließend wieder ausgespült oder mit einem Shampoo ausgewaschen wird.

## Claims

1. Use of formyl-1-methylquinolinium compounds with the following formula I, 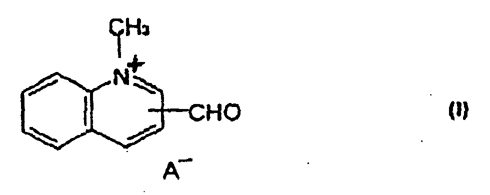 in which the formyl group CHO is bonded in the 2 or 4 position and A⁻ is a p-toluenesulphonate ion, in combination with at least one compound with primary or secondary amino group or hydroxy group chosen from primary or secondary aromatic amines chosen from the group consisting of
N-(2-hydroxyethyl)-N-ethyl-, N-(2-methoxyethyl)-, 2,3-, 2,4-, 2,5-dichloro-p-phenylenediamine, 2-chloro-p-phenylenediamine, N,N-bis(2-hydroxyethyl)-p-phenylenediamine, 2,5-dihydroxy-4-morpholinoaniline dihydrobromide, 2-, 3-, 4-aminophenol, o-, m-, p-phenylenediamine, 2,4-diaminophenoxyethanol, 2-(2,5-diaminophenyl)ethanol, 2,5-diaminotoluene, -phenol, -phenethol, 4-methylamino-, 3-amino-4-(2'-hydroxyethyloxy)-, 3,4-methylenediamino-, 3,4-methylenedioxyaniline, 3-amino-2,4-dichloro-, 4-methylamino-, 2-methyl-5-amino-, 3-methyl-4-amino-, 2-methyl-5-(2-hydroxyethylamino)-, 2-methyl-5-amino-4-chloro-,-6-methyl-3-amino-2-chloro, 5-(2-hydroxyethylamino)-4-methoxy-2-methyl-, 4-amino-2-aminomethylphenol, 2-hydroxymethyl-4-aminophenol, 2-diethylaminomethyl-4-aminophenol, 2-dimethylaminomethyl-4-aminophenol, 2,6-dichloro-4-aminophenol, 1,3-diamino-2,4-dimethoxybenzene, 2-, 3-, 4-aminobenzoic acid, -phenylacetic acid, 2,3-, 2,4-, 2,5-, 3,4-, 3,5-diaminobenzoic acid, 4-, 5-aminosalicylic acid, 3-amino-4-hydroxy-, 4-amino-3-hydroxy-benzoic acid, 2-, 3-, 4-aminobenzenesulphonic acid, 3-amino-4-hydroxybenzenesulphonic acid, 4-amino-3-hydroxynaphthalenesulphonic acid, 6-amino-7-hydroxynaphthalene-2-sulphonic acid, 7-amino-4-hydroxynaphthalene-2-sulphonic acid, 4-amino-5-hydroxynaphthalene-2,7-disulphonic acid, 3-amino-2-naphthoic acid, 3-aminophthalic acid, 5-aminoisophthalic acid, 1,3,5-, 1,2,4-triaminobenzene, 1,2,4,5-tetraaminobenzene tetrahydrochloride, 2,4,5-triaminophenol trihydrochloride, pentaaminobenzene pentahydrochloride, hexaaminobenzene hexahydrochloride, 2,4,6-triaminoresorcinol trihydrochloride, 4,5-diaminopyrocatechol sulphate, 4,6-diaminopyrogallol dihydrochloride, 3,5-diamino-4-hydroxypyrocatechol sulphate, 1,3-dimethyl-2,5-diaminobenzene, 4-amino-1-hydroxy-2-(2'-hydroxyethylaminomethyl)benzene, 1-hydroxy-2-amino-5-methylbenzene, 1-hydroxy-2-amino-6-methylbenzene, 5-amino-4-fluoro-2-methylphenol, 2,4-diamino-5-fluorotoluene, 2,4-diamino-5-(2'-hydroxyethoxy)toluene, 2,4-diamino-5-methylphenetol, 3,5-diamino-2-methoxy-1-methylbenzene, 2-amino-4-(2'-hydroxyethylamino) anisole, 2,6-bis(2'-hydroxyethyl)-1-methylbenzene, N,N'-bis(4-aminophenyl)-1,4-diazacycloheptane, 1-(2-hydroxy-5-aminobenzyl)-2-imidazolidinone, 4-amino-2-((4-[(5-amino-2-hydroxyphenyl)methyl] piperazinyl)methyl)phenol, 4-nitroaniline, 4-nitro-1,3-phenylenediamine, 4-amino-2-nitrodiphenylamine-2'-carboxylic acid, 1-amino-5-chloro-4-(2-hydroxyethylamino)-2-nitrobenzene, aromatic anilines or phenols with a further aromatic radical, such as 4,4'-diaminostilbene dihydrochloride, 4,4'-diaminostilbene-2,2'-disulphonic acid, Na salt, 4,4'-diaminodiphenylmethane, -sulphide, -sulphoxide, - amine, 4,4'-diaminodiphenylamine-2-sulphonic acid, 4,4'-diaminobenzophenone, -diphenyl ether, 3,3',4,4'-tetraaminodiphenyl tetrahydrochloride, 3,3',4,4'-tetraaminobenzophenone, 1,3-bis(2,4-diaminophenoxy)propane tetrahydrochloride, 1,8-bis(2,5-diaminophenoxy)-3,6-dioxaoctane tetrahydrochloride, 1,3-bis(4-aminophenylamino)propane, -2-propanol, 1,3-bis[N-(4-aminophenyl)-2-hydroxyethylamino]-2-propanol, N,N-bis[2-(4-aminophenoxy)ethyl]methylamine trihydrochloride, bis(2-hydroxy-5-aminophenyl)methane,
nitrogen-containing heterocyclic compounds chosen from the group consisting of
2-, 3-, 4-amino-, 2-amino-3-hydroxy-, 2,6-diamino-, 2,5-diamino-, 2,3-diamino-, 2-dimethylamino-5-amino-, 3-amino-2-methylamino-6-methoxy-, 2,3-diamino-6-methoxy-, 3,5-diamino-2,6-dimethoxy-, 2,4,5-triamino-, 2,6-dihydroxy-3,4-dimethylpyridine, 4,5,6-triamino-, 2-hydroxy-4,5,6-triamino-, 4-hydroxy-2,5,6-triamino-, 2,4,5,6-tetraamino-, 2-methylamino-4,5,6-triamino-, 2,4-, 4,5-diamino-, 2-amino-4-methoxy-6-methylpyrimidine, 2,3,4-trimethylpyrrole, 2,4-dimethyl-3-ethylpyrrole, 3,5-diaminopyrazole, -1,2,4-triazole, 3-amino-, 3-amino-5-hydroxypyrazole, 2-,3-, 8-aminoquinoline, 4-aminoquinaldine, 2-, 6-aminonicotinic acid, 5-aminoisoquinoline; 5-, 6-aminoindazole, 5-, 7-aminobenzimidazole, -benzothiazole, 2,5-dihydroxy-4-morpholinoaniline, 1-phenyl-3-methylpyrazolone-5, 4,5-diamino-1-(2-hydroxyethyl)pyrazole, 2-(aminoethylamino)-5-aminopyridine, (2((2,4-diaminophenyl)amino)ethyl-5-amino(2-pyridyl))amine, (2((4-aminophenyl)amino)ethyl-5-amino(2-pyridyl))-amine and indole and indoleine derivatives, such as 4-, 5-, 6-, 7-aminoindole, 4-, 5-, 6-, 7-hydroxyindole, 5,6-dihydroxyindole, 5,6-dihydroxyindoleine and 4-hydroxyindoleine, and in each case of the physiologically compatible salts of these compounds formed with preferably inorganic acids,
and aromatic hydroxy compounds, and/or at least one CH-active compound for dyeing keratin-containing fibres.

2. Agent for dyeing keratin-containing fibres, in particular human hair, which comprises formyl-1-methylquinolinium compounds with the following formula I, 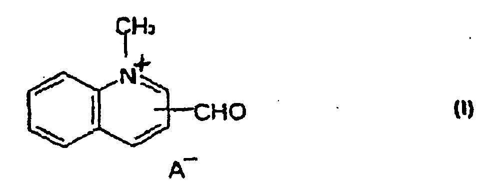 in which the formyl group CHO is bonded in the 2 or 4 position and A⁻ is a p-toluenesulphonate ion, in combination with at least one compound with primary or secondary amino group or hydroxy group chosen from primary or secondary aromatic amines chosen from the group consisting of
N-(2-hydroxyethyl)-N-ethyl-, N-(2-methoxyethyl)-, 2,3-, 2,4-, 2,5-dichloro-p-phenylenediamine, 2-chloro-p-phenylenediamine, N,N-bis(2-hydroxyethyl)-p-phenylenediamine, 2,5-dihydroxy-4-morpholinoaniline dihydrobromide, 2-, 3-, 4-aminophenol, o-, m-, p-phenylenediamine, 2,4-diaminophenoxyethanol, 2-(2,5-diaminophenyl)ethanol; 2,5-diaminotoluenine, -phenol, -phenethol, 4-methylamino-, 3-amino-4-(2'-hydroxyethyloxy)-, 3,4-methylenediamino-, 3,4-methylenedioxyaniline, 3-amino-2,4-dichloro-, 4-methylamino-, 2-methyl-5-amino-, 3-methyl-4-amino-, 2-methyl-5-(2-hydroxyethylamino)-. 2-methyl-5-amino-4-chloro-, 6-methyl-3-amino-2-chloro, 5-(2-hydroxyethylamino)-4-methoxy-2-methyl-, 4-amino-2-aminomethylphenol, 2-hydroxymethyl-4-aminophenol, 2-diethylaminomethyl-4-aminophenol, 2-dimethylaminomethyl-4-aminophenol, 2,6-dichloro-4-aminophenol, 1,3-diamino-2,4-dimethoxybenzene, 2-, 3-, 4-aminobenzoic acid, -phenylacetic acid, 2,3-, 2,4-, 2,5-, 3,4-, 3,5-diaminobenzoic acid, 4-, 5-aminosalicylic acid, 3-amino-4-hydroxy-, 4-amino-3-hydroxybenzoic acid, 2-, 3-, 4-aminobenzenesulphonic acid, 3-amino-4-hydroxybenzenesulphonic acid, 4-amino-3-hydroxynaphthalenesulphonic acid, 6-amino-7-hydroxynaphthalene-2-sulphonic acid, 7-amino-4-hydroxynaphthalene-2-sulphonic acid, 4-amino-5-hydroxynaphthalene-2,7-disulphonic acid, 3-amino-2-naphthoic acid, 3-aminophthalic acid, 5-aminoisophthalic acid, 1,3,5-, 1,2,4-triaminobenzene, 1,2,4,5-tetraaminobenzene tetrahydrochloride, 2,4,5-triaminophenol trihydrochloride, pentaaminobenzene pentahydrochloride, hexaaminobenzene hexahydrochloride, 2,4,6-triaminoresorcinol trihydrochloride, 4,5-diaminopyrocatechol sulphate, 4,6-diaminopyrogallol dihydrochloride, 3,5-diamino-4-hydroxypyrocatechol sulphate, 1,3-dimethyl-2,5-diaminobenzene, 4-amino-1-hydroxy-2-(2'-hydroxyethylaminomethyl)benzene, 1-hydroxy-2-amino-5-methylbenzene, 1-hydroxy-2-amino-6-methylbenzene, 5-amino-4-fluoro-2-methylphenol, 2,4-diamino-5-fluorotoluene, 2,4-diamino-5-(2'-hydroxyethoxy)toluene, 2,4-diamino-5-methylphenetol, 3,5-diamino-2-methoxy-1-methylbenzene, 2-amino-4-(2'-hydroxyethylamino)anisole, 2,6-bis(2'-hydroxyethyl)-1-methylbenzene, N,N'-bis(4-aminophenyl)-1,4-diazacycloheptane, 1-(2-hydroxy-5-aminobenzyl)-2-imidazolidinone, 4-amino-2-((4-[(5-amino-2-hydroxyphenyl)methyl]piperazinyl)methyl)phenol, 4-nitroaniline, 4-nitro-1,3-phenylenediamine, 4-amino-2-nitrodiphenylamine-2'-carboxylic acid, 1-amino-5-chloro-4-(2-hydroxyethylamino)-2-nitrobenzene, aromatic anilines or phenols with a further aromatic radical, such as 4,4'-diaminostilbene dihydrochloride, 4,4'-diaminostilbene-2,2'-disulphonic acid, Na salt, 4,4'-diaminodiphenylmethane, -sulphide, -sulphoxide, -amine, 4,4'-diaminodiphenylamine-2-sulphonic acid, 4,4'-diaminobenzophenone, -diphenyl ether, 3,3',4,4'-tetraaminodiphenyl tetrahydrochloride, 3,3',4,4'-tetraaminobenzophenone, 1,3-bis(2,4-diaminophenoxy)propane tetrahydrochloride, 1,8-bis(2,5-diaminophenoxy)-3,6-dioxaoctane tetrahydrochloride, 1,3-bis(4-aminophenylamino)propane, -2-propanol, 1,3-bis[N-(4-aminophenyl)-2-hydroxyethylamino]-2-propanol, N,N-bis[2-(4-aminophenoxy)ethyl]methylamine trihydrochloride, bis(2-hydroxy-5-aminophenyl)methane,
nitrogen-containing heterocyclic compounds chosen from the group consisting of
2-, 3-, 4-amino-, 2-amino-3-hydroxy-, 2,6-diamino-, 2,5-diamino-, 2,3-diamino-, 2-dimethylamino-5-amino-, 3-amino-2-methylamino-6-methoxy-, 2,3-diamino-6-methoxy-, 3,5-diamino-2,6-dimethoxy-, 2,4,5-triamino-, 2,6-dihydroxy-3,4-dimethylpyridine, 4,5,6-triamino-, 2-hydroxy-4,5,6-triamino-, 4-hydroxy-2,5,6-triamino-, 2,4,-5,6-tetraamino-, 2-methylamino-4,5,6-triamino-, 2,4-, 4,5-diamino-, 2-amino-4-methoxy-6-methylpyrimidine, 2,3,4-trimethylpyrrole, 2,4-dimethyl-3-ethylpyrrole, 3,5-diaminopyrazole, -1,2,4-triazole, 3-amino-, 3-amino-5-hydroxypyrazole, 2-,3-, 8-aminoquinoline, 4-aminoquinaldine, 2-, 6-aminonicotinic acid, 5-aminoisoquinoline, 5-, 6-aminoindazole, 5-, 7-aminobenzimidazole, -benzothiazole, 2,5-dihydroxy-4-morpholinoaniline, 1-phenyl-3-methylpyrazolone-5, 4,5-diamino-1-(2-hydroxyethyl)pyrazole, 2-(aminoethylamino)-5-aminopyridine; (2((2,4-diaminophenyl)amino)ethyl-5-amino(2-pyridyl))amine, (2((4-aminophenyl)amino)ethyl-5-amino (2-pyridyl))amine and indole and indoleine derivatives, such as 4-, 5-, 6-, 7-aminoindole, 4-, 5-, 6-, 7-hydroxyindole, 5,6-dihydroxyindole, 5,6-dihydroxyindoleine and 4-hydroxyindoleine, and in each case of the physiologically compatible salts of these compounds formed with preferably inorganic acids,
and aromatic hydroxy compounds, and/or at least one CH-active compound.

3. Agent according to Claim 2, **characterized in that** the compounds according to formula (I) are used in the form of their hydrates.

4. Agent according to one of Claims 2 or 3, **characterized in that** the compounds with the formula I are present in an amount of in each case 0.03 to 65 mmol, in particular 1 to 40 mmol, in each case based on 100 g of the total dyeing agent.

5. Agent according to Claim 2, **characterized in that** the aromatic hydroxy compounds are chosen from the group consisting of 2-, 4-, 5-methylresorcinol, 2,5-dimethylresorcinol, resorcinol, 3-methoxyphenol, pyrocatechol, hydroquinone, pyrogallol, phloroglucine, hydroxyhydroquinone, 2-, 3-, 4-methoxy-, 3-dimethylamino-, 2-(2-hydroxyethyl)-, 3,4-methylenedioxyphenol, 2,4-, 3,4-dihydroxybenzoic acid, -phenylacetic acid, gallic acid, 2,4,6-trihydroxybenzoic acid, -acetophenone, 2-, 4-chlororesorcinol, 1-naphthol, 1,5-, 2,3-, 2,7-dihydroxynaphthalene, 6-dimethylamino-4-hydroxy-2-naphthalenesulphonic acid, 3,6-dihydroxy-2,7-naphthalenesulphonic acid.

6. Agent according to one of Claims 2 or 5, **characterized in that** the CH-active compounds are chosen from the group consisting of 1,2,3,3-tetramethyl-3H-indoleium iodide, 1,2,3,3-tetramethyl-3H-indoleium p-toluenesulphonate, 1,2,3,3-tetramethyl-3H-indoleium methanesulphonate, Fischer's base (1,3,3-trimethyl-2-methyleneindoline), 2,3-dimethylbenzothiazolium iodide, 2,3-dimethylbenzothiazolium p-toluene sulphonate, 1,2-dimethylnaphtho[1,2-d]thiazomlium p-toluenesulphonate, 1-ethyl-2-methylnaphtho[1,2-d] thiazolium p-toluenesulphonate, rhodanine, rhodanine-3-acetic acid, 1-ethyl-2-quinaldinium iodide, 1,4-dimethylquinolinium iodide, 1-methyl-2-quinaldinium iodide, barbituric acid, thiobarbituric acid, 1,3-dimethylthiobarbituric acid, 1,3-diethylthiobarbituric acid, diethylthiobarbituric acid, oxindole, 3-indoxyl acetate, coumaranone and 1-methyl-3-phenyl-2-pyrazolinone.

7. Agent according to Claim 2, **characterized in that** the further compound is chosen from the group consisting of N,N-bis(2-hydroxyethyl)-p-phenylenediamine, 2,5-diaminotoluene, 2-(2,5-diaminophenyl)ethanol, 2-aminophenol, 3-aminophenol, 4-aminophenol, 2-aminomethyl-4-aminophenol, 2-(diethylaminomethyl)-4-aminophenol., 1-hydroxy-2-amino-5-methylbenzene, 4-amino-3-methylphenol, 2,4-diaminophenoxyethanol, 3-amino-2,4-dichlorophenol, 2-methyl-5-aminophenol, 2-methyl-5-(2-hydroxyethylamino)phenol, 6-methyl-3-amino-2-chlorophenol, 2-methyl-5-amino-4-chlorophenol, 3,4-methylenedioxyaniline, 3,4-diaminobenzoic acid, 3,5-diamino-2-methoxy-1-methylbenzene, 2-amino-4-(2'-hydroxyethylamino) anisole, 2,6-bis(2'-hydroxyethyl)-1-methylbenzene, 1,3-bis(2,4-diaminophenoxy) propane, 4,5-diamino-1-(2-hydroxyethyl)pyrazole, N,N'-bis(4-aminophenyl)-1,4-diazacycloheptane, bis(2-hydroxy-5-aminophenyl) methane, 1,8-bis(2,5-diaminophenoxy)-3,6-dioxaoctane, 4,4'-diaminodiphenylamine, 2-amino-3-hydroxypyridine, 3-amino-2-methylamino-6-methoxypyridine, 2,6-dihydroxy-3,4-dimethylpyridine, 3,5-diamino-2, 6-dimethoxypyridine, 5,6-dihydroxyindole, 5,6-dihydroxyindoline, 2,4,5,6-tetraaminopyrimidine, 2-hydroxy-4,5,6-triaminopyrimidine, 4-hydroxy-2,5,6-triaminopyrimidine and in each case of the physiologically compatible salts of these compounds formed preferably with inorganic acids.

8. Agent according to one of Claims 2 to 7, **characterized in that** it comprises colour enhancers chosen from the group consisting of piperidine, piperidine-2-carboxylic acid, piperidine-3-carboxylic acid, piperidine-4-carboxylic acid, pyridine, 2-hydroxypyridine, 3-hydroxypyridine, 4-hydroxypyridine, imidazole, 1-methylimidazole, histidine, pyrroleidine, proline, pyrroleidone, pyrroleidone-5-carboxylic acid, pyrazole, 1,2,4-triazole, piperazidine or any mixtures thereof.

9. Agent according to one of Claims 2 to 8,
**characterized in that** it comprises direct dyes from the group of nitrophenylenediamines, nitroaminophenols, anthraquinones or indophenols, preferably in an amount of from 0.01 to 20% by weight, based on the total dyeing agent.

10. Agent according to one of Claims 2 to 9, **characterized in that** ammonium or metal salts chosen from the group of formates, carbonates, halides, sulphates, butyrates; valerates, caproates, acetates, lactates, glycolates, tartrates, citrates, gluconates, propionates, phosphates and phosphonates of alkali metals, such as potassium, sodium or lithium, alkali earth metals, such as magnesium, calcium, strontium or barium, or of aluminium, manganese, iron, cobalt, copper or zinc are added.

11. Agent according to one of Claims 2 to 10, **characterized in that** oxidizing agents are used in an amount of from 0.01 to 6% by weight, based on the application solution.

12. Agent according to one of Claims 2 to 11, **characterized in that** the oxidizing agent used is H₂O₂.

13. Agent according to one of Claims 2 to 12, **characterized in that** anionic, zwitterionic and/or nonionic surfactants are used.

14. Method of dyeing keratin-containing fibres, in which a dyeing agent according to one of Claims 2 to 13 for dyeing keratin-containing fibres, and customary cosmetic ingredients is applied to the keratin-containing fibres, left on the fibres for a certain amount of time, usually about 30 minutes, and then rinsed out again or washed out with a shampoo.

## Revendications

1. Utilisation de composés de formyl-1-méthylquinolinium répondant à la formule suivante I 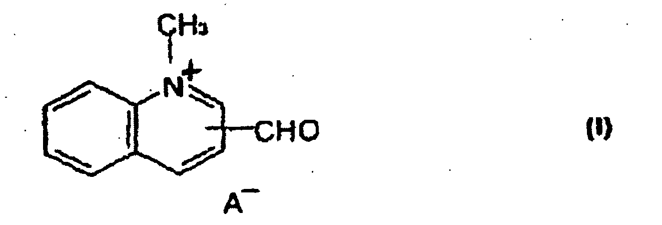 dans laquelle le groupe formyle CHO est lié en position 2 ou en position 4 et A- représente un ion de p-toluènesulfonate, en combinaison avec au moins un composé comprenant un groupe hydroxyle ou un groupe amino primaire ou secondaire, choisi parmi des amines aromatiques primaires ou secondaires choisies parmi le groupe constitué par :
la N-(2-hydroxyéthyl)-N-éthyl-, la N-(2-méthoxyéthyl)-, la 2,3-, la 2,4-, la 2,5-dichloro-p-phénylènediamine, la 2-chloro-p-phénylènediamine, la N,N-bis-(2-hydroxyéthyl)-p-phénylènediamine, le dibromhydrate de la 2,5-dihydroxy-4-morpholinoaniline, le 2-, le 3-, le 4-aminophénol, la o-, la m-, la p-phénylènediamine, le 2,4-diaminophénoxyéthanol, le 2-(2,5-diaminophényl)éthanol, le 2,5-diaminotoluène, -phénol, -phénéthol, la 4-méthylamino-, la 3-amino-4-(2'-hydroxyéthyloxy)-, la 3,4-méthylènediamino-, la 3,4-méthylènedioxy-aniline, le 3-amino-2,4-dichloro-, le 4-méthylamino-, le 2-méthyl-5-amino-, le 3-méthyl-4-amino-, le 2-méthyl-5-(2-hydroxyéthylamino)-, le 2-méthyl-5-amino-4-chloro-, le 6-méthyl-3-amino-2-chloro-, le 5-(2-hydroxyéthylamino)-4-méthoxy-2-méthyl-, le 4-amino-2-aminométhylphénol, le 2-hydroxyméthyl-4-aminophénol, le 2-diéthylaminométhyl-4-aminophénol, le 2-diméthylaminométhyl-4-aminophénol, le 2,6-dichloro-4-aminophénol, le 1,3-diamino-2,4-diméthoxybenzène, l'acide 2-, l'acide 3-, l'acide 4-aminobenzoïque, l'acide 2-, l'acide 3-, l'acide 4-aminophénylacétique, l'acide 2,3-, l'acide 2,4-, l'acide 2,5-, l'acide 3,4-, l'acide 3,5-diaminobenzoïque, l'acide 4-, l'acide 5-aminosalicylique, l'acide 3-amino-4-hydroxy-, l'acide 4-amino-3-hydroxybenzoïque, l'acide 2-, l'acide 3-, l'acide 4-aminobenzènesulfonique, l'acide 3-amino-4-hydroxybenzènesulfonique, l'acide 4-amino-3-hydroxynaphtalène-sulfonique, l'acide 6-amino-7-hydroxynaphtalène-2-sulfonique, l'acide 7-amino-4-hydroxy-naphtalène-2-sulfonique, l'acide 4-amino-5-hydroxynaphtalène-2,7-disulfonique, l'acide 3-amino-2-naphtoïque, l'acide 3-aminophtalique, l'acide 5-aminoisophtalique, le 1,3,5-, le 1,2,4-triaminobenzène, le tétrachlorhydrate de 1,2,4,5-tétra-amino-benzène, le trichlorhydrate de 2,4,5-triaminophénol, le pentachlorhydrate de pentaaminobenzène, l'hexachlorhydrate d'hexaaminobenzène, le trichlorhydrate de 2,4,6-triaminorésorcinol, le sulfate de 4,5-diaminopyrocatéchol, le dichlorhydrate de 4,6-diaminopyrogallol, le sulfate de 3,5-diamino-4-hydroxypyrocatéchol, le 1,3-diméthyl-2,5 diaminobenzène, le 4-amino-1-hydroxy-2-(2'-hydroxyéthylaminométhyl)benzène, le 1-hydroxy-2-amino-5-méthyl-benzène, le 1-hydroxy-2-amino-6-méthylbenzène, le 5-amino-4-fluoro-2-méthylphénol, le 2,4-diamino-5-fluorotoluène, le 2,4-diamino-5-(2'-hydroxyéthoxy)toluène, le 2,4-diamino-5-méthylphénéthol, le 3,5-diamino-2-méthoxy-1-méthylbenzène, le 2-amino-4-(2'-hydroxyéthylamino)anisole, le 2,6-bis-(2'-hydroxyéthyl)-1-méthylbenzène, le N,N'-bis(4-aminophényl)-1,4-diazacycloheptane, la 1-(2-hydroxy-5-aminobenzyl)-2-imidazolidinone, le 4-amino-2-{(4-[(5-amino-2-hydroxyphényl)-méthyl]pipérazinyl)méthyl}phénol, la 4-nitroaniline, la 4-nitro-1,3-phénylènediamine, l'acide 4-amino-2-nitrodiphénylamine-2'-carboxylique, le 1-amino-5-chloro-4-(2-hydroxyéthylamino)-2-nitrobenzène, des anilines aromatiques, respectivement des phénols contenant un radical aromatique supplémentaire tel que le dichlorhydrate de 4,4'-diaminostilbène, le sel de sodium de l'acide 4,4'-diaminostilbène-2,2'-disulfonique, le 4,4'-diaminodiphénylméthane, le 4,4'-diaminodiphénylsulfure, le 4,4'-diaminodiphénylsulfoxyde, la 4,4'-diaminodiphénylamine, l'acide 4,4'-diaminodiphénylamine-2-sulfonique, la 4,4'-diaminobenzophénone, le 4,4'-diaminodiphényléther, le tétrachlorhydrate de 3,3',4,4'-tétra-aminodiphényle, la 3,3',4,4'-tétraamiriobenzophénone, le tétrachlorhydrate de 1,3-bis-(2,4-diamino-phénoxy)propane, le tétrachlorhydrate de 1,8-bis-(2,5-diaminophénoxy)-3,6-dioxaoctane, le 1,3-bis-(4-aminophényl-amino)propane, le 1,3-bis-(4-aminophénylamino)-2-propanol, le 1,3-bis-[N-(4-aminophényl)-2-hydroxyéthylamino]-2-propanol, le trichlorhydrate de la N,N-bis-[2-(4-aminophénoxy)-éthyl]méthylamine, le bis-(2-hydroxy-5-aminophényl)méthane,
des composés hétérocycliques azotés choisis parmi le groupe constitué par la 2-, la 3-, la 4-amino-, la 2-amino-3-hydroxy-, la 2,6-diamino-, la 2,5-diamino-, la 2,3-diamino-, la 2-diméthylamino-5-amino-, la 3-amino-2-méthylamino-6-méthoxy-, la 2,3-diamino-6-méthoxy-, la 3,5-diamino-2,6-diméthoxy-, la 2,4,5-triamino-, la 2,6-dihydroxy-3,4-diméthylpyridine, la 4,5,6-triamino-, la 2-hydroxy-4,5,6-triamino-, la 4-hydroxy-2,5,6-triamino-, la 2,4,5,6-tétraamino-, la 2-méthylamino-4,5,6-triamino-, la 2,4-, la 4,5-diamino-, la 2-amino-4-méthoxy-6-méthylpyrimidine, le 2,3,4-triméthylpyrrole, le 2,4-diméthyl-3-éthylpyrrole, le 3,5-diamino-pyrazole, -1,2,4-triazole, le 3-amino-, le 3-amino-5-hydroxypyrazole, la 2-, la 3-, la 8-aminoquinoléine, la 4-aminoquinaldine, l'acide 2-, l'acide 6-aminonicotinique, la 5-aminoisoquinoléine, le 5-, le 6-amirioindazole, le 5-, le 7-aminobenzimidazole, -benzothiazole, la 2,5-dihydroxy-4-morpholinoaniline, la 1-phényl-3-méthyl-pyrazolone-5, le 4,5-diamino-1-(2-hydroxyéthyl)pyrazole, la 2-(amino-éthylamino)-5-aminopyridine, la (2((2,4-diaminophényl)amino)-éthyl-5-amino-(2-pyridyl))amine, la (2(4-aminophényl)amino)-éthyl-5-amino-(2-pyridyl))amine, ainsi que des dérivés d'indole et d'indoline, tels que le 4-,le 5-, le 6-, le 7-aminoindole, le 4-, le 5-, le 6-, le 7-hydroxyindole, le 5,6-dihydroxyindole, la 5,6-dihydroxyindoline et la 4-hydroxyindoline, ainsi que respectivement par les sels physiologiquement acceptables de ces composés, formés avec des acides de préférence inorganiques,
et des composés hydroxylés aromatiques, et/ou au moins un composé CH-actif pour la teinture de fibres kératiniques.

2. Agent pour la teinture de fibres kératiniques, en particulier de cheveux humains qui contient des composés de formyl-1-méthylquinolinium répondant à la formule suivante 1 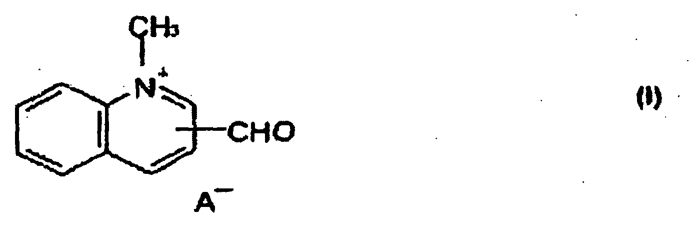 dans laquelle le groupe formyle CHO est lié en position 2 ou en position 4 et A' représente un ion de p-toluènesulfonate,
en combinaison avec au moins un composé comprenant un groupe hydroxyle ou un groupe amino primaire ou secondaire, choisi parmi des amines aromatiques primaires ou secondaires choisies parmi le groupe constitué par :
la N-(2-hydroxyéthyl)-N-éthyl-, la N-(2-méthoxyéthyl)-, la 2,3-, la 2,4-, la 2,5-dichloro-p-phénylènediamine, la 2-chloro-p-phénylènediamine, la N,N-bis-(2-hydroxyéthyl)-p-phénylènediamine, le dibromhydrate de la 2,5-dihydroxy-4-morpholinoaniline, le 2-, le 3-, le 4-aminophénol, la o-, la m-, la p-phénylènediamine, le 2,4-diaminophénoxyéthanol, le 2-(2,5-diaminophényl)éthanol, le 2,5-diamino-toluène, -phénol, -phénéthol, la 4-méthylamino-, la 3-amino-4-(2'-hydroxyéthyloxy)-, la 3,4-méthylènediamino-, la 3,4-méthylènedioxyaniline, le 3-amino-2,4-dichloro-, le 4-méthylamino-, le 2-méthyl-5-amino-, le 3-méthyl-4-amino-, le 2-méthyl-5-(2-hydroxyéthylamino)-, le 2-méthyl-5-amino-4-chloro-, le 6-méthyl-3-amino-2-chloro-, le 5-(2-hydroxyéthylamino)-4-méthoxy-2-méthyl-, le 4-amino-2-aminométhylphénol, le 2-hydroxyméthyl-4-aminophénol, le 2-diéthylaminométhyl-4-aminophénol, le 2-diméthylaminométhyl-4-aminophénol, le 2,6-dichloro-4-aminophénol, le 1,3-diamino-2,4-diméthoxybenzène, l'acide 2-, l'acide 3-, l'acide 4-aminobenzoïque, l'acide 2-, l'acide 3-, l'acide 4-aminophénylacétique, l'acide 2,3-, l'acide 2,4-, l'acide 2,5-, l'acide 3,4-, l'acide 3,5-diaminobenzoïque, l'acide 4-, l'acide 5-aminosalicylique, l'acide 3-amino-4-hydroxy-, l'acide 4-amino-3-hydroxybenzoïque, l'acide 2-, l'acide 3-, l'acide 4-aminobenzènesulfonique, l'acide 3-amino-4-hydroxybenzènesulfonique, l'acide 4-amino-3-hydroxynaphtalène-sulfonique, l'acide 6-amino-7-hydroxynaphtalène-2-sulfonique, l'acide 7-amino-4-hydroxy-naphtalène-2-sulfonique, l'acide 4-amino-5-hydroxynaphtalène-2,7-disulfonique, l'acide 3-amino-2-naphtoïque, l'acide 3-aminophtalique, l'acide 5-aminoisophtalique, le 1,3,5-, le 1,2,4-triaminobenzène, le tétrachlorhydrate de 1,2,4,5-tétra-amino-benzène, le trichlorhydrate de 2,4,5-triaminophénol, le pentachlorhydrate de pentaaminobenzène, l'hexachlorhydrate d'hexaaminobenzène, le trichlorhydrate de 2,4,6-triaminorésorcinol, le sulfate de 4,5-diaminopyrocatéchol, le dichlorhydrate de 4,6-diaminopyrogallol, le sulfate de 3,5-diamino-4-hydroxypyrocatéchol, le 1,3-diméthyl-2,5 diaminobenzène, le 4-amino-1-hydroxy-2-(2'-hydroxyéthylaminométhyl)benzène, le 1-hydroxy-2-amino-5-méthylbenzène, le 1-hydroxy-2-amino-6-méthylbenzène, le 5-amino-4-fluoro-2-méthylphénol, le 2,4-diamino-5-fluorotoluène, le 2,4-diamino-5-(2'-hydroxyéthoxy)toluène, le 2,4-diamino-5-méthylphénéthol, le 3,5-diamino-2-méthoxy-1-méthylbenzène, le 2-amino-4-(2'-hydroxyéthylamino)anisole, le 2,6-bis-(2'-hydroxyéthyl)-1-méthylbenzène, le N,N'-bis(4-aminophényl)-1,4-diazacycloheptane, la 1-(2-hydroxy-5-aminobenzyl)-2-imidazolidinone, le 4-amino-2-{(4-[(5-amino-2-hydroxyphényl)-méthyl]pipérazinyl)méthyl}phénol, la 4-nitroaniline, la 4-nitro-1,3-phénylènediamine, l'acide 4-amino-2-nitrodiphénylamine-2'-carboxylique, le 1-amino-5-chloro-4-(2-hydroxyéthylamino)-2-nitrobenzène, des anilines aromatiques, respectivement des phénols contenant un radical aromatique supplémentaire tel que le dichlorhydrate de 4,4'-diaminostilbène, le sel de sodium de l'acide 4,4'-diaminostilbène-2,2'-disulfonique, le 4,4'-diaminodiphénylméthane, le 4,4'-diaminodiphénylsulfure, le 4,4'-diaminodiphénylsulfoxyde, la 4,4'-diaminodiphénylamine, l'acide 4,4'-diaminodiphénylamine-2-sulfonique, la 4,4'-diamino-benzophénone, le 4,4'-diaminodiphényléther, le tétrachlorhydrate de 3,3',4,4'-tétraaminodiphényle, la 3,3',4,4'-tétraaminobenzophénone, le tétrachlorhydrate de 1,3-bis-(2,4-diamino-phénoxy)propane, le tétrachlorhydrate de 1,8-bis-(2,5-diamino-phénoxy)-3,6-dioxaoctane, le 1,3-bis-(4-aminophénylamino)propane, le 1,3-bis-(4-aminophénylamino)-2-propanol, le 1,3-bis-[N-(4-aminophényl)-2-hydroxyéthylamino]-2-propanol, le trichlorhydrate de la N,N-bis-[2-(4-aminophénoxy)-éthyl]méthylamine, le bis-(2-hydroxy-5-aminophényl)méthane,
des composés hétérocycliques azotés choisis parmi le groupe constitué par la 2-, la 3-, la 4-amino-, la 2-amino-3-hydroxy-, la 2,6-diamino-, la 2,5-diamino-, la 2,3-diamino-, la 2-diméthylamino-5-amino-, la 3-amino-2-méthylamino-6-méthoxy-, la 2,3-diamino-6-méthoxy-, la 3,5-diamino-2,6-diméthoxy-, la 2,4,5-triamino-, la 2,6-dihydroxy-3,4-diméthylpyridine, la 4,5,6-triamino-, la 2-hydroxy-4,5,6-triamino-, la 4-hydroxy-2,5,6-triamino-, la 2,4,5,6-tétraamino-, la 2-méthylamino-4,5,6-triamino-, la 2,4-, la 4,5-diamino-, la 2-amino-4-méthoxy-6-méthylpyrimidine, le 2,3,4-triméthylpyrrole, le 2,4-diméthyl-3-éthylpyrrole, le 3,5-diamino-pyrazole, -1,2,4-triazole, le 3-amino-, le 3-amino-5-hydroxypyrazole, la 2-, la 3-, la 8-aminoquinoléine, la 4-aminoquinaldine, l'acide 2-, l'acide 6-aminonicotinique, la 5-aminoisoquinoléine, le 5-, le 6-aminoindazole, le 5-, le 7-aminobenzimidazole, -benzothiazole, la 2,5-dihydroxy-4-morpholinoaniline, la 1-phényl-3-méthylpyrazolone-5, le 4,5-diamino-1-(2-hydroxyéthyl)-pyrazole, la 2-(amino-éthylamino)-5-aminopyridine, la (2((2,4-diaminophényl)amino)-éthyl-5-amino-(2-pyridyl))amine, la (2(,4-aminophényl)amino)-éthyl-5-amino-(2-pyridyl))amine, ainsi que des dérivés d'indole et d'indoline, tels que le 4-, le 5-, le 6-, le 7-aminoindole, le 4-, le 5-, le 6-, le 7-hydroxyindole, le 5,6-dihydroxyindole, la 5,6-dihydroxyindoline et la 4-hydroxyindoline, ainsi que respectivement par les sels physiologiquement acceptables de ces composés, formés avec des acides de préférence inorganiques,
et des composés hydroxylés aromatiques, et/ou au moins un composé CH-actif.

3. Agent selon la revendication 2, **caractérisé en ce qu'**on met en oeuvre les composés répondant à la formule (I) sous la forme de leurs hydrates.

4. Agent selon l'une quelconque des revendications 2 ou 3, **caractérisé en ce que** les composés répondant à la formule I sont contenus en une quantité respectivement de 0,03 à 65, en particulier de 1 à 40 millimoles, chaque fois rapportée à 100 g de la teinture totale.

5. Agent selon la revendication 2, **caractérisé en ce que** les composés hydroxylés aromatiques sont choisis parmi le groupe constitué par le 2-, le 4-, le 5-méthylrésorcinol, le 2,5-diméthylrésorcinol, le résorcinol, le 3-méthoxyphénol, le pyrocatéchol, l'hydroquinone, le pyrogallol, la phloroglucine, l'hydroxyhydroquinone, le 2-, le 3-, le 4-méthoxy-, le 3-diméthylamino-, le 2-(2-hydroxyéthyl)-, le 3,4-méthylènedioxyphénol, l'acide 2,4-, l'acide 3,4-dihydroxybenzoïque, -phénylacétique, l'acide gallique, l'acide 2,4,6-trihydroxybenzoïque, la 2,4,6-trihydroxy-acétophénone, le 2-, le 4-chlororésorcinol, le 1-naphtol, le 1,5-, le 2,3-, le 2,7-dihydroxynaphtalène, l'acide 6-diméthylamino-4-hydroxy-2-naphtalènesulfonique, l'acide 3,6-dihydroxy-2,7-naphtalènesulfonique.

6. Agent selon l'une quelconque des revendications 2 ou 5, **caractérisé en ce que** les composés CH-actifs sont choisis parmi le groupe constitué par l'iodure de 1,2,3,3-tétraméthyl-3H-indolium, le p-toluènesulfonate de 1,2,3,3-tétraméthyl-3H-indolium, le méthanesulfonate de 1,2,3,3-tétraméthyl-3H-indolium, la base de Fischer (1,3,3-triméthyl-2-méthylèneindoline), l'iodure de 2,3-diméthylbenzothiazolium, le p-toluènesulfonate de 2,3-diméthylbenzothiazolium, le p-toluènesulfonate de 1,2-diméthyl-naphto[1,2-d]thiazolium, le p-toluènesulfonate de 1-éthyl-2-méthyl-naphto[1,2-d]thiazolium, la rhodanine, l'acide rhodanine-3-acétique, l'iodure de 1-éthyl-2-quinaldinium, l'iodure de 1,4-diméthylquinolinium, l'iodure de 1-méthyl-2-quinaldinium, l'acide barbiturique, l'acide thiobarbiturique, l'acide 1,3-diméthylthiobarbiturique, l'acide 1,3-diéthylthiobarbiturique, l'acide diéthylthiobarbiturique, l'oxindole, le 3-indoxylacétate, la coumaranone et la 1-méthyl-3-phényl-2-pyrazolinone.

7. Agent selon la revendication 2, **caractérisé en ce que** le composé supplémentaire est choisi parmi le groupe constitué par la N,N-bis-(2-hydroxyéthyl)-p-phénylènediamine, le 2,5-diaminotoluène, le 2-(2,5-diaminophényl)éthanol, le 2-aminophénol, le 3-aminophénol, le 4-aminophénol, le 2-aminométhyl-4-aminophénol, le 2-(diéthylaminométhyl)-4-aminophénol, le 1-hydroxy-2-amino-5-méthylbenzène, le 4-amino-3-méthylphénol, le 2,4-diaminophénoxyéthanol, le 3-amino-2,4-dichlorophénol, le 2-méthyl-5-aminophénol, le 2-méthyl-5-(2-hydroxyéthylamino)phénol, le 6-méthyl-3-amino-2-chlorophénol, le 2-méthyl-5-amino-4-chlorophénol, la 3,4-méthylènedioxyaniline, l'acide 3,4-diaminobenzoïque, le 3,5-diamino-2-méthoxy-1-méthylbenzène, le 2-amino-4-(2'-hydroxyéthylamino)anisole, le 2,6-bis-(2'-hydroxyéthyl)-1-méthylbenzène, le 1,3-bis-(2,4-diaminophénoxy)propane, le 4,5-diamino-1-(2-hydroxyéthyl)pyrazole, le N,N'-bis(4-aminophényl)-1,4-diazacycloheptane, le bis-(2-hydroxy-5-aminophényl)méthane, le 1,8-bis-(2,5-diaminophénoxy)-3,6-dioxaoctane, la 4,4'-diaminodiphénylamine, la 2-amino-3-hydroxypyridine, la 3-amino-2-méthylamino-6-méthoxypyridine, le 2,6-dihydroxy-3,4-diméthylpyridine, la 3,5-diamino-2,6-diméthoxypyridine, le 5,6-dihydroxyindole, la 5,6-dihydroxyindoline, la 2,4,5,6-tétra-aminopyrimidine, la 2-hydroxy-4,5,6-triaminopyrimidine, la 4-hydroxy-2,5,6-triaminopyrimidine, ainsi que respectivement par les sels physiologiquement acceptables de ces composés, formés de préférence avec des acides inorganiques.

8. Agent selon l'une quelconque des revendications 2 à 7, **caractérisé en ce qu'**il contient des renforçateurs de colorants choisis parmi le groupe constitué par la pipéridine, l'acide pipéridine-2-carboxylique, l'acide pipéridine-3-carboxylique, l'acide pipéridine-4-carboxylique, la pyridine, la 2-hydroxypyridine, la 3-hydroxypyridine, la 4-hydroxypyridine, l'imidazole, le 1-méthylimidazole, l'histidine, la pyrrolidine, la proline, la pyrrolidone, l'acide pyrrolidone-5-carboxylique, le pyrazole, le 1,2,4-triazole, la pipérazidine ou l'un quelconque de leurs mélanges.

9. Agent selon l'une quelconque des revendications 2 à 8, **caractérisé en ce qu'**il contient des colorants montant directement sur la fibre choisis parmi le groupe des nitrophénylènediamines, des nitroaminophénols, des anthraquinones ou des indophénols, de préférence en une quantité de 0,01 à 20 % en poids rapportés à la teinture totale.

10. Agent selon l'une quelconque des revendications 2 à 9, **caractérisé en ce qu'**on ajoute des sels d'ammonium ou des sets métalliques choisis parmi le groupe des formiates, des carbonates, des halogénures, des sulfates, des butyrates, des valérates, des caproates, des acétates, des lactates, des glycollates, des tartrates, des citrates, des gluconates, des propionates, des phosphates et des phosphonates de métaux alcalins tels que le potassium, le sodium ou le lithium, de métaux alcalino-terreux tels que le magnésium, le calcium, le strontium ou le baryum ou de l'aluminium, du manganèse, du fer, du cobalt, du cuivre ou du zinc.

11. Agent selon l'une quelconque des revendications 2 à 10, **caractérisé en ce qu'**il contient des agents d'oxydation en une quantité de 0,01 à 6 % en poids, rapportés à la solution d'utilisation.

12. Agent selon l'une quelconque des revendications 2 à 11, **caractérisé en ce que** l'on met en oeuvre du H₂O₂ à titre d'agent d'oxydation.

13. Agent selon l'une quelconque des revendications 2 à 12, **caractérisé en ce qu'**il contient des agents tensioactifs anioniques, zwitterioniques et/ou non ioniques.

14. Procédé pour la teinture de fibres kératiniques, dans lequel on applique une teinture, selon l'une quelconque des revendications 2 à 13, pour la teinture de fibres kératiniques ainsi que des ingrédients cosmétiques usuels, sur les fibres kératiniques, on les laisse sur les fibres pendant un certain temps, habituellement pendant environ 30 minutes, avant de les éliminer par rinçage ou par lavage avec un shampooing.
